Europäisches Patentamt
European Patent Office
Office européen des brevets

(19) (11) Veröffentlichungsnummer: **0 019 089**
A1

# EUROPÄISCHE PATENTANMELDUNG

(12)

(21) Anmeldenummer: 80102000.9

(22) Anmeldetag: 14.04.80

(51) Int. Cl.³: C 07 C 69/753
C 07 C 69/757, C 07 C 69/65
C 07 D 307/32, C 07 D 303/02
C 07 D 303/08, C 07 C 47/232
C 07 C 47/24, C 07 D 317/52
C 07 C 21/24, C 07 C 25/24

(30) Priorität: 23.04.79 DE 2916401

(43) Veröffentlichungstag der Anmeldung: 26.11.80 Patentblatt 80/24

(84) Benannte Vertragsstaaten: BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Jautelat, Manfred, Dr.
Muellersbaum 28
D-5093 Burscheid 1(DE)

(72) Erfinder: Arlt, Dieter, Dr.
Rybniker Strasse 2
D-5000 Köln 80(DE)

(72) Erfinder: Lantzsch, Reinhard, Dr.
Heymannstrasse 32
D-5090 Leverkusen 1(DE)

(72) Erfinder: Fuchs, Rainer, Dr.
Roeberstrasse 8
D-5600 Wuppertal(DE)

(72) Erfinder: Riebel, Hans-Jochem, Dr.
In der Beek 92
D-5600 Wuppertal 1(DE)

(72) Erfinder: Schröder, Rolf, Dr.
Pahlkestrasse 17
D-5600 Wuppertal 1(DE)

(72) Erfinder: Harnisch, Horst, Dr.
Heinenbusch 4
D-5203 Much(DE)

(54) **Verfahren zur Herstellung von 3-(2-Arylvinyl)-2,2-dimethyl-cyclopropan-1-carbonsäureestern, neue Zwischenprodukte dafür und deren Herstellung.**

(57) Die vorliegende Erfindung betrifft ein Verfahren zur Herstellung von 3-(2-Arylvinyl)-2, 2-dimethylcyclopropan-1-carbonsäureestern der allgemeinen Formel (I)

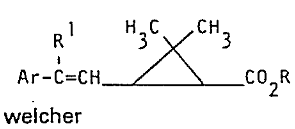

(I)

in welcher

Ar, R, R¹ die in der Beschreibung angegebene Bedeutung besitzen, das dadurch gekennzeichnet ist, daß man Verbindungen der allgemeinen Formel (II)

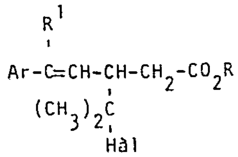

(II)

mit einer Base umsetzt sowie neue Zwischemprodukte zu ihrer Herstellung und Verfahren zur Herstellung dieser Zwischenprodukte.

EP 0 019 089 A1

BAYER AKTIENGESELLSCHAFT 5090 Leverkusen, Bayerwerk
Zentralbereich
Patente, Marken und Lizenzen Rt-kl
IV b

BEZEICHNUNG GEÄNDERT
siehe Titelseite

Verfahren zur Herstellung von 3-(2-Arylvinyl)-2,2-dimethyl-cyclopropan-1-carbonsäureestern und neue Zwischenprodukte dafür

Die vorliegende Erfindung betrifft ein neues Verfahren zur Herstellung von 3-(2-Arylvinyl)-2,2-dimethyl-cyclopropan-1-carbonsäureestern und Zwischenprodukte zur Durchführung dieses Verfahrens sowie deren Herstellung.

Es ist bereits bekannt geworden, daß bestimmte Ester von 3-Styryl-2,2-dimethylcyclopropancarbonsäuren insektizide Eigenschaften besitzen (Deutsche Offenlegungsschrift 2 738 150). Es ist jedoch noch keine wirtschaftliche Synthese für Carbonsäuren dieser Art in technischem Maßstab bekannt.

Die Knüpfung der C-C-Doppelbindungen der Styrylgruppe erfolgt durch eine Wittigreaktion bei der Butyllithium als Base verwendet wird und unter Schutzgas bei -78°C gearbeitet werden muß. Dieser Syntheseweg ist somit für eine technische Herstellung nicht gangbar.

Ferner ist auch keine für eine Herstellung im größeren Maßstab verwendbare Synthese für den 2,2-Dimethyl-3-formyl-1-carbonsäureester bekannt.

1. Es wurde nun gefunden, daß 3-(Arylvinyl)-2,2-dimethyl-cyclopropan-1-carbonsäureester der allgemeinen Formel (I)

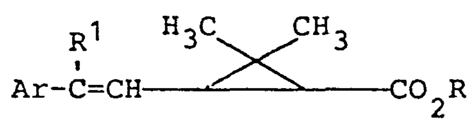 (I)

in welcher

Ar für substituierte oder unsubstituierte carbocyclische oder heterocyclische aromatische Reste steht und

R für Alkyl oder für einen bei Pyrethroiden üblichen Alkoholrest steht, und

$R^1$ für Wasserstoff, Fluor oder Chlor steht,

erhalten werden, indem man Verbindungen der allgemeinen Formel (II)

$$\begin{array}{l} \quad\quad R^1 \\ \quad\quad | \\ Ar-C=CH-CH-CH_2-CO_2R \\ \quad\quad\quad\quad\quad | \\ \quad\quad (CH_3)_2C \\ \quad\quad\quad\quad\quad | \\ \quad\quad\quad\quad\quad Hal \end{array} \qquad (II)$$

in welcher

Ar, $R^1$ und R die oben angegebene Bedeutung besitzen und

Hal für Chlor oder Brom steht,

mit einer gegebenenfalls wäßrigen Base gegebenenfalls in Gegenwart eines Verdünnungsmittels in Gegenwart eines Phasentransferkatalysators umsetzt.

2. Es wurde ferner gefunden, daß die Verbindungen der Formel (I) bevorzugt entstehen, wenn nicht zu starke Basen verwendet werden und wenn in Gegenwart eines Phasentransferkatalysators gearbeitet wird.

3. Es wurden ferner die neuen Verbindungen der allgemeinen Formel (II) gefunden, in welcher Ar, $R^1$, R und Hal die oben angegebene Bedeutung besitzen.

4. Es wurde ferner ein Verfahren zur Herstellung der neuen Verbindungen der allgemeinen Formel (II) gefunden, dadurch gekennzeichnet, daß Verbindungen der allgemeinen Formel (III)

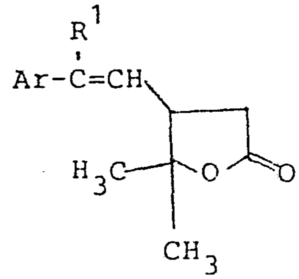

(III)

in welcher

Ar und $R^1$ die unter 1. (oben) angegebene Bedeutung haben,

mit einem Halogenierungsmittel sowie gleichzeitig oder nacheinander mit einem Alkohol der Formel (IV)

R-OH (IV)

in welcher

R die unter 1. (oben) angegebene Bedeutung besitzt,

gegebenenfalls in Gegenwart eines Verdünnungsmittels und gegebenenfalls in Gegenwart von gasförmigem Halogenwasserstoff, umgesetzt werden.

5. Es wurden ferner die neuen Verbindungen der Formel (III) gefunden, in welcher Ar und $R^1$ die unter 1. (oben) angegebene Bedeutung haben.

6. Es wurde ferner ein Verfahren zur Herstellung der neuen Verbindungen der Formel (III) gefunden, dadurch gekennzeichnet, daß man

a) Verbindungen der Formel (V)

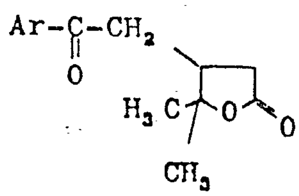

(V)

in welcher

Ar die oben angegebene Bedeutung hat,

mit Phosphorpentachlorid umsetzt oder

b) Verbindungen der Formel (VI)

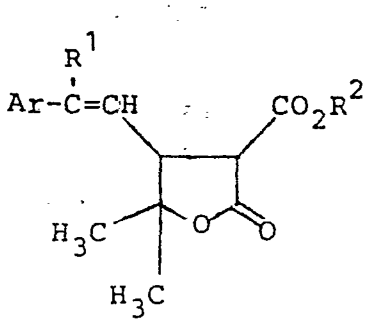

(VI)

in welcher

Ar und $R^1$ die unter 1. (oben) angegebene Bedeutung haben und

$R^2$ $C_{1-4}$-Alkyl bedeutet,

decarbalkoxyliert oder verseift und decarboxyliert oder

c) Verbindungen der Formel (V)

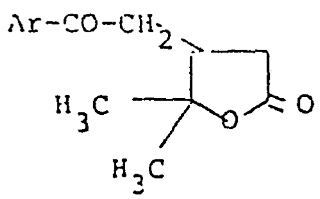

(V)

in welcher Ar die oben angegebene Bedeutung hat, reduziert und Wasser abspaltet.

7. Es wurden ferner die neuen Verbindungen der Formel (V) gefunden, in welcher Ar die unter 1. (oben) angegebene Bedeutung besitzt, jedoch nicht unsubstituiertes Phenyl bedeutet.

8. Es wurden ferner die neuen Verbindungen der Formel (VI) gefunden, in der Ar, $R^1$ und $R^2$ die unter 6. (oben) angegebene Bedeutung haben.

9. Es wurde ferner ein Verfahren zur Herstellung von Verbindungen der allgemeinen Formel (VI) gefunden, dadurch gekennzeichnet, daß man Verbindungen der allgemeinen Formel (VII)

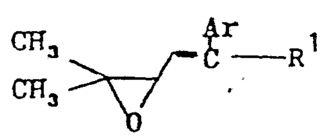

(VII)

in welcher

Ar und $R^1$ die unter 1. (oben) angegebene Bedeutung haben

mit Malonestern der Formel (VIII)

$$CH_2(CO_2R^2)_2 \qquad (VIII)$$

$R^2$ $C_{1-4}$-Alkyl bedeutet,

in Gegenwart eines Base sowie gegebenenfalls in Gegenwart eines Verdünnungsmittels umsetzt.

10. Es wurde ferner gefunden, daß die Verbindungen der Formel (VI) bevorzugt nach dem Verfahren 9 (oben) dann erhalten werden, wenn man unterhalb von 50°C arbeitet.

11. Es wurden ferner die neuen Verbindungen der Formel (VII) gefunden, in welcher Ar und $R^1$ die unter 1. (oben) angegebene Bedeutung haben.

12. Es wurde ferner ein Verfahren zur Herstellung der Verbindungen der Formel (VII) gefunden, dadurch gekennzeichnet, daß man Verbindungen der Formel (IX)

$$R^1\text{-}\overset{\text{Ar}}{\overset{|}{C}}\text{=CH-CH=C(CH}_3)_2 \qquad (IX)$$

in welcher

Ar und $R^1$ die oben angegebene Bedeutung haben,

oxidiert.

13. Es wurden ferner die neuen Verbindungen der Formel (IX) gefunden, in welcher Ar und $R^1$ die unter 1. (oben) angegebene Bedeutung haben.

14. Es wurde ferner ein Verfahren zur Herstellung der Verbindungen IX gefunden, dadurch gekennzeichnet, daß man

a) Verbindungen der Formel X A oder X B

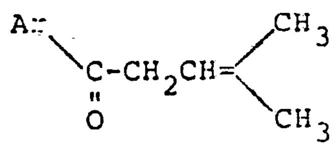

(X A)

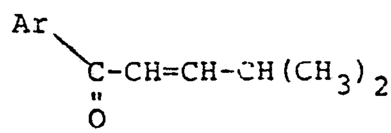

(X B)

in welcher

Ar die unter 1. (oben) angegebene Bedeutung besitzt,

oder ein Gemisch derselben, mit Phosphorpentachlorid und anschließend einer Base, umsetzt oder

b) Verbindungen der Formel (XI)

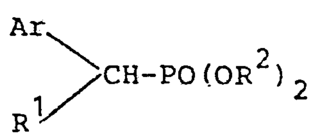

(XI)

in welcher

Ar, $R^1$ und $R^2$ die unter 6. (oben) angegebene Bedeutung haben,

mit Dimethylacrolein der Formel (XII)

$(CH_3)_2C=CH-CHO$ (XII)

umsetzt oder

c) Verbindungen der Formel (XVII)

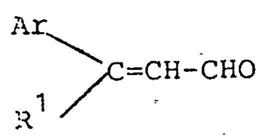
(XVII)

in welcher

Ar und $R^1$ die unter 1. (oben) angegebene Bedeutung haben,

mit der Verbindung der Formel (XVIII)

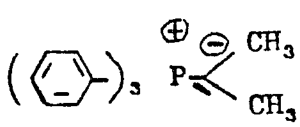
(XVIII)

umsetzt

oder

d) Verbindungen der Formel (XVII) mit Isopropylmagnesiumchlorid der Formel (XIX)

$$(i\text{-}C_3H_7\text{-}MgCl) \qquad (XIX)$$

umsetzt.

15. Es wurde ferner ein Verfahren zur Herstellung der teilweise bekannten Verbindungen der allgemeinen Formel (XVII)

$$\begin{array}{c} R^1 \\ | \\ Ar\text{-}C\text{=}CH\text{-}CHO \end{array}$$

in welcher

Ar und $R^1$ die unter 1. (oben) angegebene Bedeutung haben,

dadurch gekennzeichnet, daß man Acetophenone der allgemeinen Formel (XVI)

$$Ar\text{-}CO\text{-}CH_3 \qquad (XVI)$$

in welcher

Ar die unter 1. (oben) angegebene Bedeutung hat,

ohne Zusatz eines Verdünnungsmittels mit $POCl_3$ und Dimethylformamid umsetzt und die Reaktionsprodukte nach Hydrolyse ohne destillative Aufarbeitung isoliert.

16. Es wurde ferner ein Verfahren zur Herstellung bestimmter Verbindungen der Formel (V) die sich unter der Formel (V A) zusammenfassen lassen

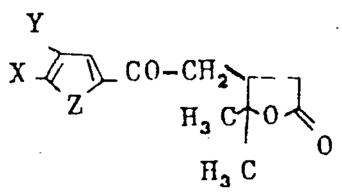

(V A)

in welcher

X für Wasserstoff, $C_{1-4}$-Alkyl, Halogen, Aryl, Aralkyl, Aryloxy, Arylthio, $C_{1-4}$-Alkoxy, $C_{1-4}$-Alkylthio steht,

Y für Wasserstoff steht oder gleich X ist, oder

X und Y für Methylendioxy oder Ethylendioxy stehen und

Z für O, S oder -CH=CH- steht,

gefunden, das dadurch gekennzeichnet ist, daß man die Verbindung der Formel (XX)

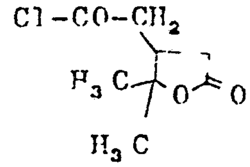

(XX)

mit Verbindungen der Formel (XXI)

in welcher

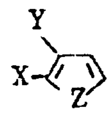

(XXI)

X, Y und Z die oben angegebene Bedeutung haben,

in Gegenwart eines Friedel-Crafts-Katalysators und gegebenenfalls in Gegenwart eines Verdünnungsmittels umsetzt.

17. Es wurde ferner die neue Verbindung der Formel (XX) gefunden.

18. Es wurde ferner ein Verfahren zur Herstellung der Verbindung (XX) gefunden, dadurch gekennzeichnet, daß die Verbindung der Formel (XXII)

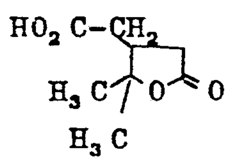

(XXII)

mit einem Chlorierungsmittel umgesetzt wird.

Die erfindungsgemäßen Verfahren unter Verwendung der erfindungsgemäßen Zwischen- und Endprodukte dienen zur Herstellung unter anderem der aus DE-OS 2 738 150 bekannten Verbindungen. Sie sind einfacher und technisch leichter durchführbar als das aus DE-OS 2 738 150 bekannte Verfahren zur Herstellung dieser Verbindungen.

Verwendet man beim Verfahren 1 (oben) 5-(4'-Chlorphenyl)-5-chlor-3-(1'-chlor-1'-methyl)-ethyl-pent-4-en-säureethylester als Ausgangsstoff, kann der Reaktionsablauf durch folgendes Formelschema wiedergegeben werden:

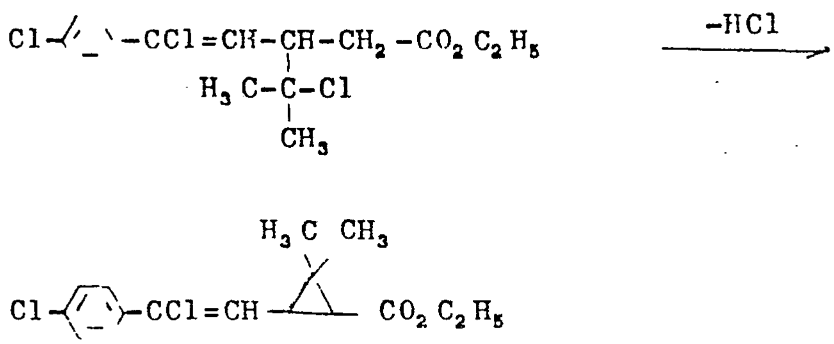


Die beim Verfahren 1 verwendbaren Ausgangsstoffe sind durch die allgemeine Formel (II) definiert. Darin steht Ar bevorzugt für gegebenenfalls durch X substituierte Phenyl-, Naphthyl-, Furan- oder Thiophen-Reste, wobei X für Halogen, Cyano, Nitro, Aryl, Aralkyl, Aryloxy, Arylthio, $C_{1-4}$-Alkyl, $C_{1-4}$-Alkoxy, $C_{1-4}$-Alkylthio, $C_{1-4}$-Halogenalkyl, $C_{1-4}$-Halogenalkoxy, $C_{1-4}$-Halogenalkylthio, gegebenenfalls halogensubstituiertes $C_{1-4}$-Alkylsulfonyl, Dialkylamino mit 1 oder 2 Kohlenstoffatomen in jedem Alkylsubstituenten, gegebenenfalls halogensubstituiertes Methylendioxy oder Ethylendioxy steht und $R^1$ für Chlor steht.

Besonders bevorzugt steht Ar für einen gegebenenfalls durch einen oder mehrere gleiche oder verschiedene Substituenten X substituierten Phenylrest, wobei X bevorzugt für Fluor, Chlor, Brom, Cyano, Nitro, Methyl, Ethyl, Propyl, Isopropyl, tert.-Butyl, Methoxy, Ethoxy, Methylmercapto, Trifluormethyl, Trifluormethoxy, Difluormethoxy, Difluorchlormethoxy, Tetrafluorethoxy, Trifluorchlorethoxy, Trifluormethylmercapto, Methylsulfonyl, Trifluormethylsulfonyl, Methylendioxy, Difluormethylendioxy Ethylendioxy, Trifluorethylendioxy steht, ferner steht Ar bevorzugt für einen Naphthylrest oder einen gegebenenfalls durch Chlor oder Brom substituierten Furan- oder Thiophenrest.

R steht bevorzugt für $C_{1-4}$-Alkyl oder für einen gegebenenfalls durch Halogen oder Cyan substituierten 3-Phenoxybenzylrest;

Ganz besonders bevorzugt steht Ar für einen gegebenenfalls durch einen oder zwei gleiche oder verschiedene Substituenten X in 3- oder 4-Stellung substituierten Phenylrest, wobei X bevorzugt für Fluor, Chlor, Brom, Methyl, Methoxy, Ethoxy, Methylmercapto, Trifluormethyl, Trifluormethoxy, Trifluormethylmercapto, Methylendioxy, Difluormethylendioxy oder Trifluorethylendioxy steht oder für einen Naphthylrest,

R steht besonders bevorzugt für Methyl oder Ethyl.

$R^1$ steht ganz besonders bevorzugt für Chlor.

Verfahren 1 wird durchgeführt, indem man die Verbindungen der Formel II gegebenenfalls in einem Verdünnungsmittel löst, den Katalysator hinzufügt und anschließend die Base hinzugibt. Als Verdünnungsmittel kommen beispielsweise Kohlenwasserstoffe wie Benzin, Petrolether, Benzol, Toluol, Xylol, Cyclohexan, Chlorkohlenwasserstoffe wie Methylenchlorid, Chlorbenzol, Dichlorbenzol oder Nitrile wie Acetonitril in Frage.

Ganz allgemein können alle inerten, nicht mit Wasser mischbaren Verdünnungsmittel verwendet werden.

Als Basen werden bevorzugt wäßrige Basen wie NaOH oder KOH verwendet, wobei der Wassergehalt sehr gering sein kann. So ist es beispielsweise auch möglich technisches, pulverisiertes Kaliumhydroxyd ohne weiteren Wasserzusatz zu verwenden. Ferner können auch Natrium- oder Kaliumcarbonat, insbesondere unter Verwendung von Acetonitril als Base verwendet werden.

Das Verfahren wird bevorzugt in Gegenwart eines Phasentransferkatalysators durchgeführt.

Als Phasentransferkatalysator kommen beispielsweise Kronenether, Tetraalkylphosphoniumsalze oder bevorzugt Tetraalkylammoniumsalze in Frage. Als ganz besonders bevorzugte Katalysatoren seien genannt:

Tetrapropylammoniumbromid, Tetrabutylammoniumchlorid und -bromid, Benzyltriethylammoniumchlorid, Methyltrioctylammoniumchlorid.

Die Katalysatormenge kann in weiten Grenzen schwanken. Im allgemeinen haben sich 0,1 - 15 Gew.-%, vorzugsweise 0,3 bis 10 Gew.-%, bezogen auf das Gewicht der eingesetzten Verbindungen der allgemeinen Formel (I) bewährt.

Das Verfahren wird bei Temperaturen zwischen $-20^{o}C$ und $80^{o}C$, bevorzugt zwischen 0 und $50^{o}C$, durchgeführt. Bei höheren Temperaturen wird ein zusätzliches Molekül Chlorwasserstoff abgespalten und man erhält nicht die gewünschten Verbindungen der allgemeinen Formel (I).

Es wird bevorzugt unter Normaldruck gearbeitet.

Die Verwendung von billigen wäßrigen Basen ist ein entscheidender Vorteil, da ähnliche Reaktionen sonst nur mit starken, wasserfreien Basen durchgeführt werden können, wie beispielsweise Natriumhydrid, Kalium-tert.-Butylat, Natrium-tert.-Amylat (US-P 3 652 652; US-P 3 077 496). Natriumhydrid ist im technischen

Maßstab nur schwierig zu handhaben. Zudem liefern solche Basen bei ähnlichen Reaktionen nur unbefriedigende Ausbeuten.

Die Ergebnisse sind jedoch beim Verfahren 1 in den Fällen in denen $R^1$ für Fluor oder Chlor steht noch schlechter, da sich mit solch starken Basen aus der ß-(Fluor)-bzw. (chlor)-ß-aryl-vinyl-Gruppe, bedingt durch die Arylgruppe, sehr leicht Halogenwasserstoff abspalten läßt.

Aufgabe des erfindungsgemäßen Verfahrens war es daher, Basen zu finden, die eine solche Abspaltung unter bestimmten Bedingungen nicht bewirken. Dies ist überraschenderweise mit den erfindungsgemäßen Basen und bei den erfindungsgemäßen Reaktionstemperaturen nicht der Fall. Zudem ist es als äußerst überraschend anzusehen, daß auch bei Verwendung wäßriger Basen praktisch keine Verseifung der Estergruppe auftritt, die ja bekanntlich im alkalischen Medium unter sehr milden Bedingungen verläuft.

Nach Verfahren 1. lassen sich bevorzugt folgende Cyclopropancarbonsäureester der Formel (I) herstellen:

2,2-Dimethyl-3-[2'-chlor-2'-(4'-fluor-phenyl)-vinyl]-cyclopropan-1-carbonsäuremethyl und -ethylester
2,2-Dimethyl-3-[2'-chlor-2'-(4'-chlor-phenyl)-vinyl]-cyclopropan-1-carbonsäuremethyl und -ethylester
2,2-Dimethyl-3-[2'-chlor-2'-(4'-brom-phenyl)-vinyl]-cyclopropan-1-carbonsäuremethyl und -ethylester

2,2-Dimethyl-3-[2'-chlor-2'-phenyl-vinyl]-cyclopropan-1-carbonsäuremethyl- und -ethylester

2,2-Dimethyl-3-[2'-chlor-2'-(3'-chlor-phenyl)-vinyl]-cyclopropan-1-carbonsäuremethyl- und -ethylester

2,2-Dimethyl-3-[2'-chlor-2'-(3',4'-dichlor)-phenyl-vinyl]-cyclopropan-1-carbonsäuremethyl- und -ethylester

2,2-Dimethyl-3-[2'-chlor-2'-(4'-methyl-phenyl)-vinyl]-cyclopropan-1-carbonsäuremethyl- und -ethylester

2,2-Dimethyl-3-[2'-chlor-2'-(3'-brom-phenyl)-vinyl]-cyclopropan-1-carbonsäuremethyl- und -ethylester

2,2-Dimethyl-3-[2'-chlor-2'-(2'-chlor-phenyl)-vinyl]-cyclopropan-1-carbonsäuremethyl- und -ethylester

2,2-Dimethyl-3-[2'-chlor-2'-(2',6'-difluor-phenyl)-vinyl]-cyclopropan-1-carbonsäuremethyl- und -ethylester

Die Verbindungen der allgemeinen Formel (II) sind neu. Sie werden nach dem unter 4. (oben) angegebenen Verfahren erhalten, indem man Verbindungen der allgemeinen Formel (III) mit einem Halogenierungsmittel sowie gleichzeitig oder nacheinander mit einem Alkohol der Formel (IV) gegebenenfalls in Gegenwart eines Verdünnungsmittels und gegebenenfalls in Gegenwart von gasförmigem Halogenwasserstoff umsetzt.

Verwendet man bei Verfahren 4. (oben) 4,4-Dimethyl-3-[2'-(4'-methyl-phenyl)-2'-(chlor)-vinyl]-$\gamma$-butyrolacton, Thionylchlorid und Ethanol als Ausgangsstoffe, kann der Reaktionsverlauf durch folgendes Formelschema wiedergegeben werden:

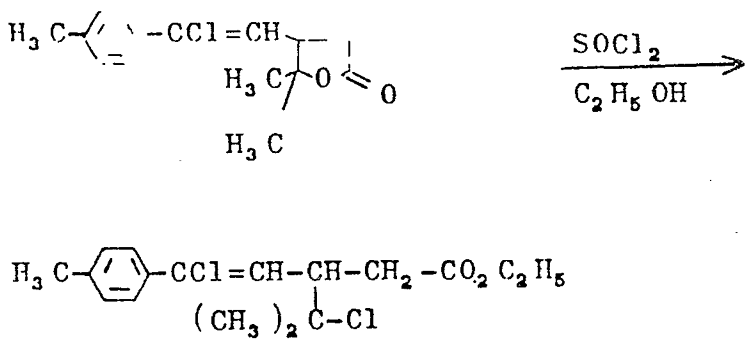


Die beim Verfahren 4 verwendbaren Ausgangsstoffe sind durch die allgemeine Formel (III) definiert. Die bevorzugten und besonders bevorzugten Substituenten Ar und $R^1$ sind die gleichen wie bei Verfahren 1. Die Verbindungen der Formel (III) sind neu; ihre Herstellung wird weiter unten beschrieben.

Folgende Halogenierungsmittel können bei Verfahren 4. Verwendung finden: Chlorwasserstoff, Bromwasserstoff, Thionylchlorid, Phosgen, Phosphortrichlorid, Phosphorpentachlorid, bevorzugt ist Thionylchlorid.

Bevorzugte Alkohole, die bei Verfahren 4. Verwendung finden können sind:

Methanol, Ethanol, Propanol, iso-Propanol, Butanol, 3-Phenoxy-benzylalkohol, 3-Phenoxy-α-cyano-benzylalkohol, 3-Phenoxy-4-fluor-α-cyano-benzylalkohol.

Ganz besonders bevorzugt sind Methanol und Ethanol.

Als Verdünnungsmittel kommen beispielsweise in Frage: Benzol, Toluol, Benzin, Petrolether, Cyclohexan, Chlorbenzol, Xylol, Chloroform.

Die Reaktionstemperatur liegt zwischen 30 und 150°C, vorzugsweise zwischen 50 und 100°C.

Die Durchführung des Verfahrens kann auf zwei grundsätzlich verschiedene Arten erfolgen.

Entweder wird der Ausgangstoff der allgemeinen Formel III in einem Alkohol der Formel IV gelöst, gegebenenfalls noch ein Verdünnungsmittel zugesetzt und anschließend das Halogenierungsmittel eingeleitet oder zugetropft.

Bevorzugt wird jedoch zunächst ohne Alkoholzusatz der Ausgangsstoff der allgemeinen Formel III mit dem Halogenierungsmittel, gegebenenfalls unter Druck und gegebenenfalls in Gegenwart eines Verdünnungsmittels erhitzt. Anschließend wird der Alkohol der allgemeinen Formel IV, gegebenenfalls in Gegenwart zusätzlichen Chlorwasserstoffs zugetropft oder zugepumpt. Das Halogenierungsmittel wird bevorzugt im Überschuß eingesetzt.

Die Isolierung der Verbindungen der allgemeinen Formel II erfolgt durch Abdestillieren des Lösungsmittels. Eine weitere Reinigung ist schwierig, aber auch nicht notwendig. Die rohen Verbindungen der Formel II können dirket für Verfahren 1 verwendet werden.

Die Verbindungen der allgemeinen Formel III sind neu.

Sie werden nach dem unter 6. (oben) angegebenen Verfahren erhalten, indem man

a) Verbindungen der Formel V mit Phosphorpentachlorid umsetzt oder

b) Verbindungen der Formel VI decarbalkoxyliert oder zur Carbonsäure verseift und decarboxyliert oder

c) Verbindungen der Formel (V) reduziert und anschließend Wasser abspaltet.

Verwendet man bei Verfahren 6 a (oben) 4,4-Dimethyl-3-phenacyl- γ -butyrolacton als Ausgangsstoff, so kann der Reaktionsverlauf durch folgendes Formelschema wiedergegeben werden:

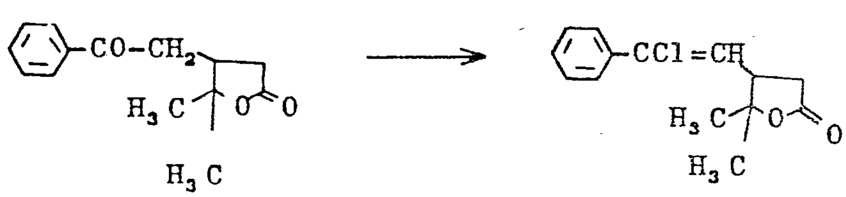

Die beim Verfahren 6 a (oben) verwendbaren Ausgangsstoffe sind durch die allgemeine Formel (V) definiert. Die bevorzugten und besonders bevorzugten Substituenten Ar sind die gleichen wie bei Verfahren 1.

Die Verbindungen der Formel (V) sind mit Ausnahme des Grundkörpers, dem 4,4-Dimethyl-3-phenacyl-γ-butyrolacton, neu. Ihre Herstellung erfolgt analog diesem nach J. Org. Chemistry, Band 39, No. 17, Seite 2604 (1974).

Ein Teil dieser Verbindungen kann jedoch auch nach einem neuen Verfahren 16 (oben) erhalten werden.

Als Chlorierungsmittel kann Phosphorpentachlorid verwendet werden.

Verfahren 6 a (oben) wird in der Weise durchgeführt, daß man die Ausgangsstoffe der Formel V mit 1 bis 2,5 Äquivalenten des Chlorierungsmittels, vorzugsweise 1,1 bis 2 Äquivalenten, umsetzt.

Vorzugsweise wird - im Gegensatz zur allgemein üblichen Arbeitsweise (Houben-Weyl, Band V, 3 Seite 912 ff) bei der Umsetzung von Ketonen mit Phosphorpentachorid - in Gegenwart eines Verdünnungsmittels gearbeitet.

Als Verdünnungsmittel kommen insbesondere Kohlenwasserstoffe wie Benzin, Petrolether, Cyclohexan, Benzol, Toluol, Chlorbenzol in Frage, aber auch Chlorkohlenwasserstoffe wie Methylenchlorid, Chloroform, Tetrachlorkohlenstoff, Dichlorethan oder Chlorbenzol, sowie Nitrile, wie Acetonitril.

Die Umsetzungstemperatur liegt zwischen $-20^{\circ}C$ und $+ 60^{\circ}C$, vorzugsweise zwischen $0^{\circ}C$ und $35^{\circ}C$.

Die Aufarbeitung erfolgt durch Rühren des Reaktionsansatzes mit Wasser, abtrennen der organischen Phase, sowie Abdestillieren des Lösungsmittels. Die Reinigung der Verbindung der allgemeinen Formel III durch Destillation oder durch Umkristallisieren.

Verwendet man bei Verfahren 6·b (oben) 4,4 -Dimethyl-3-/2'-(4'-fluorphenyl)-2'- (chlor)-vinyl7-2-ethoxycarbonyl-γ-butyrolacton als Ausgangsstoff, kann der Reaktionsverlauf durch folgendes Formelschema wiedergegeben werden:

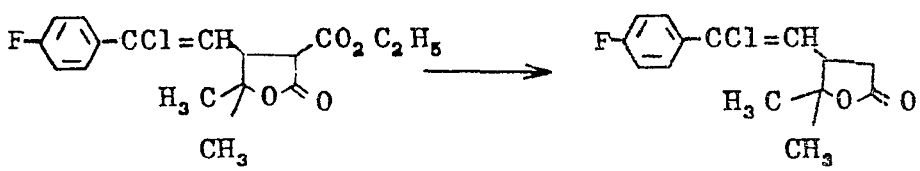

Die beim Verfahren 6 b verwendbaren Ausgangsstoffe sind durch die allgemeine Formel (VI) definiert. Die bevorzugten und besonders bevorzugten Substituenten Ar und $R^1$ sind die gleichen wie bei Verfahren 1 (oben), $R^2$ steht bevorzugt für Methyl oder Ethyl.

Die Verbindungen der Formel VI sind neu; ihre Herstellung wird weiter unten beschrieben.

Die Entfernung einer Alkoxycarbonylgruppe kann grundsätzlich im neutralen, sauren oder alkalischen Medium erfolgen. Im erfindungsgemäßen Verfahren 6 b erfolgt dies bevorzugt im neutralen oder im sauren Medium.

Hierzu werden die Verbindungen der allgemeinen Formel VI auf Temperaturen zwischen 80 und 180°C, bevorzugt auf Temperaturen zwischen 100 und 150°C, vorzugsweise in einem Verdünnungsmittel und gegebenenfalls in Gegenwart einer Säure bzw. eines sauren Katalysators erhitzt.

Als Verdünnungsmittel seien beispielsweise genannt: Wasser, Dimethylsulfoxid oder phosphorhaltige Lösungsmittel, wie sie in der DE-OS 2 638 453 beschrieben sind. Ferner kommen aber auch alle anderen inerten Lösungsmittel mit genügend hohem Siedepunkt in Betracht. Bei der Verwendung von Dimethylsulfoxid oder phosphorhaltiger Lösungsmittel wird bevorzugt unter Verwendung von Wasser und Salzen gearbeitet. Verwendet man ausschließlich Wasser als Verdünnungsmittel, so muß unter Druck gearbeitet werden und die bevorzugte Temperatur liegt oberhalb 120°C.

Bevorzugt ist jedoch das Arbeiten in Gegenwart einer Säure bzw. eines sauren Katalysators. Als solche seien beispielsweise genannt:

Schwefelsäure, Phosphorsäure, Sulfonsäuren oder saure Ionenaustauscher. Hierbei wird entweder unter Druck gearbeitet oder aber vorzugsweise der gebildete Alkohol $R^2$-OH laufend aus der Mischung, gegebenenfalls zusammen mit dem verwendeten Lösungsmittel, abdestilliert.

Die Isolierung der Verbindungen der Formel III erfolgt in üblicher Weise durch Abfiltrieren oder Extrahieren.

Die Verbindungen der allgemeinen Formel VI sind neu. Sie werden nach dem unter 9 (oben) angegebenen Verfahren erhalten, indem man Verbindungen der allgemeinen Formel VII mit Malonestern der Formel VIII in Gegenwart einer Base sowie gegebenenfalls in Gegenwart eines Verdünnungsmittels umsetzt. Verwendet man bei Verfahren 6 c (oben) 4,4-Dimethyl-3-phenacyl-8-butyrolacton als Ausgansstoff, so kann der Reaktionsverlauf durch folgendes Formelschema wiedergegeben werden:

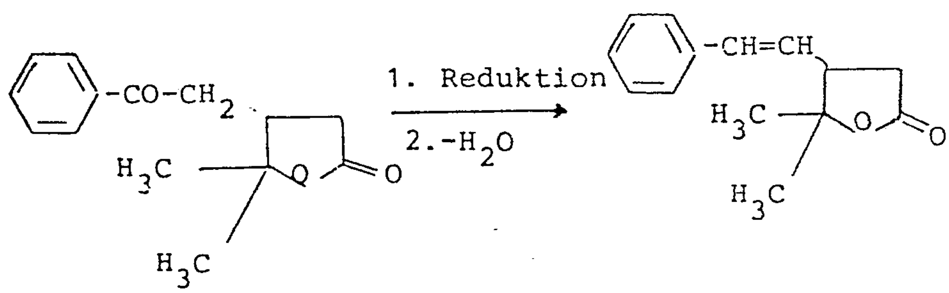


Die beim Verfahren 6c verwendbaren Ausgangsstoffe sind durch die allgemeine Formel (V) definiert. Die bevorzugten und besonders bevorzugten Substituenten Ar sind die gleichen wie bei Verfahren 1.

Erster Teilschritt des Verfahrens 6c ist eine Reduktion.

Als Reduktionsmittel kommen grundsätzlich alle Mittel in Frage, durch die ein Keton zum Alkohol reduziert wird, ohne den Lactonring anzugreifen. Beispielsweise seien genannt: Komplexe Borhydride, wie Natrium- oder Kaliumborhydrid oder Wasserstoff in Gegenwart von beispielsweise Nickel-, Palladium- oder Platin-Katalysatoren, wie beispielsweise Raney-Nickel. Die Verwendung von Natriumborhydrid ist bevorzugt.

Zweiter Teilschritt des Verfahrens 6c ist eine Dehydratisierung.

Zur Dehydratisierung werden bevorzugt saure Katalysatoren eingesetzt. Beispielsweise seien genannt: Oxalsäure, Schwefelsäure, Phosphorsäure, Kaliumhydrogensulfat, p-Toluolsulfonsäure, Aluminiumoxid, Silikate.

Außerdem kann der entstandene Alkohol acetyliert und anschließend durch Erhitzen Essigsäure abgespalten werden. Die Acetylierung erfolgt durch Acetylchlorid oder Acetanhydrid.

Verwendet man bei Verfahren 9 (oben) 2,2-Dimethyl-3-[2'-(4'-chlor-phenyl)-2'-(chlor)-vinyl]-oxiran und Malonsäuredimethylester als Ausgangsstoffe, so kann der Reaktionsverlauf durch folgendes Formelschema wiedergegeben werden:

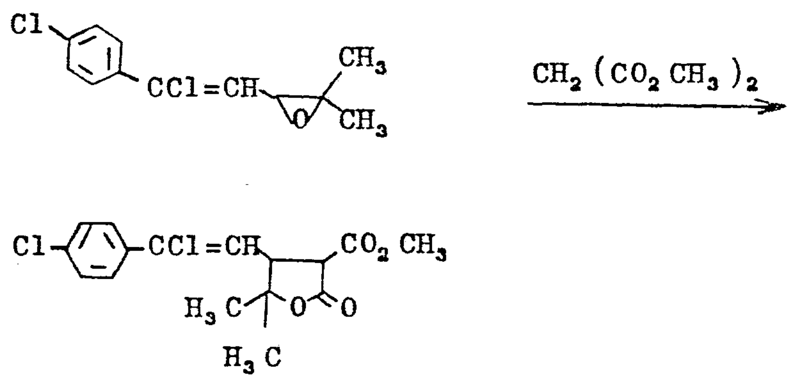


Die beim Verfahren 9 (oben) verwendbaren Ausgangsstoffe sind durch die allgemeinen Formeln VII und VIII definiert. Die Verbindungen der Formel VIII sind bekannt, die bevorzugten Substituenten $R^2$ sind Methyl und Ethyl. Die Verbindungen der allgemeinen Formel VII sind neu. Die bevorzugten und besonders bevorzugten Substituenten Ar und $R^1$ sind die gleichen wie bei Verfahren 1.

Ihre Herstellung wird weiter unten beschrieben.

Verfahren 9 wird durchgeführt, indem man zunächst das Natrium- oder Kaliumsalz des Malonesters der Formel VII herstellt, was üblicherweise mit Alkoholaten, wie Natriummethylat oder Natriumethylat durchgeführt wird. Als Verdünnungsmittel werden bevorzugt Alkohole, wie Methanol oder Ethanol, verwendet.

Zwar ist die Reaktion gemäß Verfahren 9 prinzipiell bekannt, jedoch nicht mit den verwendeten erfindungsgemäßen Substituenten. Es mußten daher Verfahrensbedingungen gefunden werden, unter denen die gegen Alkali empfindliche Aryl-halogen-vinyl-Gruppe nicht angegriffen wird.

Es wurde gefunden, daß die Verbindungen der Formel VI bevorzugt dann erhalten werden, wenn man unterhalb von 50°C arbeitet. Die bevorzugte Reaktionstemperatur liegt somit bei Verfahren 9 zwischen 0 und 50°C, besonders bevorzugt zwischen 20 und 40°C.

Das Oxiran der allgemeinen Formel VII wird bei dieser Temperatur zur Suspension des Malonestersalzes in einem Verdünnungsmittel zugetropft und die Reaktionslösung noch einige Zeit, üblicherweise etwa 1 - 6 Stunden bei der gewählten Reaktionstemperatur gehalten.

Durch Aufarbeitung in üblicher Weise kann man die Verbindung der Formel VI isolieren oder aber direkt nach Verfahren 6 b weiter umsetzen.

Die Verbindungen der allgemeinen Formel VII sind neu. Sie werden nach dem unter 12 (oben) angegebenen Verfahren erhalten, indem man Verbindungen der Formel IX oxidiert.

Verwendet man bei Verfahren 12 (oben) 1,1-Dimethyl-4 (4'-fluorphenyl)-4-chlor-1,3-butadien als Ausgangsstoff, so kann der Reaktionsverlauf durch folgendes Formelschema wiedergegeben werden:

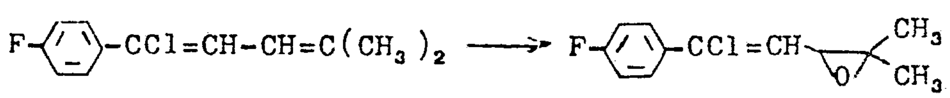

Die beim Verfahren 12 verwendbaren Ausgangsstoffe sind durch die allgemeine Formel (IX) definiert. Die bevorzugten und besonders bevorzugten Substituenten sind die gleichen wie bei Verfahren 1.

Die Verbindungen der Formel IX sind neu; ihre Herstellung wird weiter unten beschrieben.

Die überführung einer Doppelbindung in ein Oxiran ist grundsätzlich bekannt, jedoch liefert die Epoxidierung von Dienen meist nur schlechte Ausbeuten, da grundsätzlich beide Doppelbindungen angegriffen werden können.

Ferner führt die Verwendung von Persäuren sehr oft zu Folgereaktionen, da unter sauren Bedingungen Oxirane wieder geöffnet werden können.

Das gewünschte Oxiran der allgemeinen Formel (VII) wird bevorzugt dann erhalten, wenn man mit aromatischen Persäuren arbeitet. Das ist insofern überraschend, da aromatische Persäuren starke Oxidationsmittel darstellen und man annehmen mußte, daß mit diesen beide Doppelbindungen angegriffen werden, wie dies mit aliphatischen Persäuren, wie beispielsweise Perpropionsäure der Fall ist. Als Lösungsmittel für die Oxidation kommen Benzol, Methylenchlorid oder Chloroform in Frage. Gegebenenfalls kann in Gegenwart von Natriumcarbonat gearbeitet werden. Die Reaktionstemperatur liegt zwischen 0 und 50$^{o}$C, vorzugsweise bei Raumtemperatur.

Grundsätzlich können die gewünschten Oxirane auch über die Chlorhydrine mit Natriumhypochlorit und anschließende Chlorwasserstoffabspaltung erhalten werden. Doch liefert dieses Verfahren etwas schlechtere Ausbeuten als die Oxidation mit Persäuren und außerdem werden zwei statt einer Stufe benötigt.

Die Verbindungen der allgemeinen Formel (IX) sind neu. Sie werden nach dem unter 14 (oben) angegebenen Verfahren erhalten, indem man

a) Verbindungen der Formel (X A) oder (X B) oder ein Gemisch derselben mit Phosphorpentachlorid und anschließend einer Base umsetzt oder

b) Verbindungen der Formel (XI) mit Dimethylacrolein der Formel (XII) umsetzt oder

c) Verbindungen der Formel (XVII) mit Verbindungen der Formel (XVIII) umsetzt oder

d) Verbindungen der Formel (XVII) mit Isopropylmagnesiumchlorid umsetzt.

Verwendet man bei Verfahren 14 a ein Gemisch aus 2-Methyl-5-oxo-5-(4'-chlor)-phenyl-2-penten und 2-Methyl-5-oxo-5-(4'-chlor)-phenyl-3-penten als Ausgangsstoff, so kann der Reaktionsverlauf durch folgendes Formelschema wiedergegeben werden:

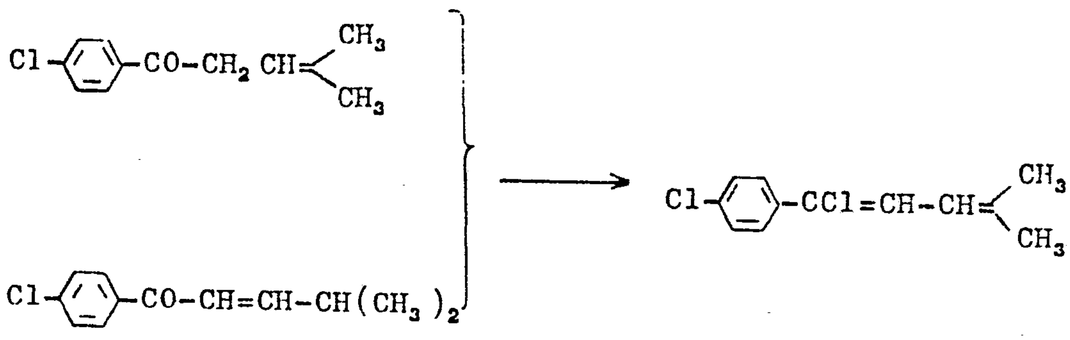

Die beim Verfahren 14 a verwendbaren Ausgangsstoffe sind durch die allgemeine Formel (X A) bzw. (X B) definiert. Die bevorzugten und besonders bevorzugten Substituenten Ar sind die gleichen wie bei Verfahren 1.

Die Verbindungen der Formel X sind teilweise bekannt. Ihre Herstellung ist in J. Org. Chemistry Band 25, Seite 272 beschrieben. Durch Kondensation der Acetophenone der Formel XVI mit Isobutyraldehyd erhält man dimere Produkte, die durch Destillation in Gegenwart von Natriumacetat in ein Monomerengemisch der Formel X A und B gespalten werden:

$$Ar{-}CO{-}CH_3 + (CH_3)_2(CH)CHO \longrightarrow \text{Dimere} \xrightarrow[NaOCOCH_3]{\Delta} X\,A + XB$$

(XVI)

Als Beispiele für die Acetophenone der Formel XVI seien genannt: Acetophenon, 4-Fluoracetophenon, 4-Chloracetophenon, 4-Bromacetophenon, 4-Methylacetophenon, 2-Acetylthiophen.

Verfahren 14 a wird erfindungsgemäß in der Weise durchgeführt, daß die Ausgangsstoffe der allgemeinen Formel X A bzw. X B bzw. Gemische von beiden in einem Verdünnungsmittel vorgelegt werden und $PCl_5$ hinzudosiert wird.

Im Gegensatz zur allgemein üblichen Arbeitsweise bei der Umsetzung von Ketonen mit Phosphorpentachlorid (Houben-Weyl, Band V, 3, Seite 912 ff) wird Verfahren 14 a bevorzugt in Gegenwart eines Verdünnungsmittels durchgeführt. Als Verdünnungsmittel kommen beispielsweise Kohlenwasserstoffe, wie Benzin, Petrolether, Pentan, Hexan, Cyclohexan, Benzol, Toluol, Xylol oder Chlorkohlenwasserstoffe wie Methylenchlorid, Tetrachlorkohlenstoff, Dichlorethan oder Chlorbenzol, sowie Nitrile, wie Acetonitril in Frage. Bevorzugt ist Toluol, Cyclohexan und Tetrachlorkohlenstoff.

Die Umsetzung eines ungesättigten Ketons mit $PCl_5$ ist praktisch immer problematisch und gibt Anlaß zu einer Vielzahl von Nebenreaktionen.

So kann beispielsweise $PCl_5$ eine Chlorierung in Allylstellung bzw. in α-Stellung zur Carbonylgruppe bewirken. Der hierbei sowie durch Eliminierung entstehende Chlorwasserstoff kann sich wieder an eine Doppelbindung addieren. Außerdem vermag $PCl_5$ auch Doppelbindungen zu chlorieren, so daß höherchlorierte Produkte entstehen.

Es wurde gefunden, daß sich diese Nebenreaktionen praktisch vollständig vermeiden lassen, wenn man in Gegenwart von Verdünnungsmitteln und bei realtiv tiefer Temperatur arbeitet sowie anschließend bei der gleichen oder bei einer niedrigeren Temperatur eine Base zusetzt, so daß das Vorhandensein von Chlorwasserstoff bei höheren Temperaturen vermieden wird.

Verfahren 14 a wird bei Temperaturen zwischen -40°C und +30°C durchgeführt, bevorzugt sind Temperaturen zwischen -10°C und +20°C.

Als Basen kommen Alkoholate, wie Natriummethylat oder Natriumethylat in Frage. Besonders bevorzugt sind wäßrige Basen, wie Natriumhydroxid, Kaliumhydroxid, Natriumcarbonat, Kaliumcarbonat in Gegenwart von Phasentransferkatalysatoren.

Als Phasentransferkatalysatoren werden bevorzugt die gleichen eingesetzt wie bei Verfahren 1.

Die Base wird in ca. der vierfachen äquimolaren Menge eingesetzt, da sie mit dem Phosphoroxichlorid reagiert

und außerdem zur Chlorwasserstoffeliminierung dient.

Die Diene der allgemeinen Formel IX können durch Destillation gereinigt werden. Falls das Dien nicht unzersetzt destillierbar ist, wird es durch sogenanntes "Andestillieren", d.h. durch längeres Erhitzen unter vermindertem Druck auf mäßig erhöhte Temperaturen, gereinigt.

Verwendet man bei Verfahren 14 b (oben) 4-Trifluormethyl-α-chlorbenzyl-O,O-diethyl-phosphonsäureester und Dimethylacrolein als Ausgangsstoffe, so kann der Reaktionsverlauf durch folgendes Formelschema wiedergegeben werden:

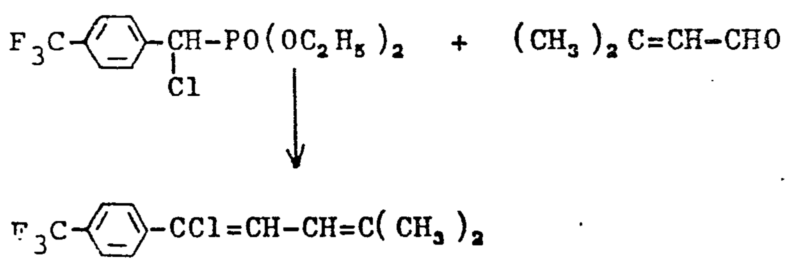

Die beim Verfahren 14 b verwendbaren Ausgangsstoffe sind durch die Formeln XI und XII definiert. Dimethylacrolein (XII) ist literaturbekannt.

Die α-Chloro-benzyl-phosphonsäureester der Formel XI sind teilweise bekannt. Sie können durch Umsetzung von α-Hydroxy-benzyl-phosphonsäureestern der Formel (XIII)

$$Ar-\underset{\displaystyle OH}{CH}-PO(OR^2)_2 \qquad (XIII)$$

in welcher

Ar und $R^2$ die unter 14 (oben) angegebene Bedeutung haben,

mit einem Chlorierungsmittel, wie z.B. Thionylchlorid oder Phosphoroxichlorid, bei Temperaturen zwischen 0 und 100°C, vorzugsweise bei 20 bis 80°C, hergestellt werden (vergleiche Chimia 28 (1974), 656 - 657; J. Am. Chem. Soc. 87 (1965), 2777 - 2778).

Die zur Herstellung der Ausgangsverbindungen der Formel XII zu verwendenden α-Hydroxy-benzyl-phosphonsäureester sind durch Formel (XIII) definiert.

Die α-Hydroxy-benzyl-phosphonsäureester der Formel (XIII) sind teilweise bekannt. Man erhält sie nach bekannten Verfahren, im allgemeinen durch Umsetzung von Aldehyden der Formel

$$Ar-CHO \qquad (XIV)$$

worin Ar die oben angegebene Bedeutung hat,

mit Phosphorigsäureester der Formel

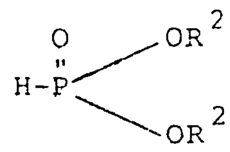

(XV)

worin $R^2$ die oben angegebene Bedeutung hat,

gegebenenfalls in Gegenwart eines Katalysators wie z.B. Triethylamin, bei Temperaturen zwischen 0 und 150°C, vorzugsweise bei 20 bis 100°C (vergleiche Houben-Weyl, Methoden der organischen Chemie, 4. Auflage (1963), Band 12/1, S. 475 - 483, Georg Thieme Verlag, Stuttgart).

Als Beispiele für die Aldehyde der Formel XIV seien genannt: Benzaldehyd, 4-Fluorbenzaldehyd, 4-Chlorbenzaldehyd, 3-Brombenzaldehyd, 4-Brombenzaldehyd, 3-Chlorbenzaldehyd, 3,4-Dichlorbenzaldehyd, 2,6-Difluorbenzaldehyd, 4-Methylbenzaldehyd, Thiophen-2-aldehyd, ß-Formyl-naphthalin, 4-Trifluormethyl-benzaldehyd.

Als Beispiele der Phosphorigsäureester der Formel XV seien genannt:

Phosphorigsäuredimethylester, Phosphorigsäurediethylester.

Verfahren 14 b ist eine Variante der Wittig-Reaktion und im Prinzip bekannt.

So erhält man beispielsweise bei der Umsetzung von Aldehyden mit γ-Chloro-benzylphosphonsäureestern in Gegenwart einer Base Chloro-styrylverbindungen (Chima 28, S. 656 - 657; 1974 und JACS 87, S. 2777 - 2778; 1965).

Als Base wird hierzu nach dem Stand der Technik Natriumhydrid verwendet, welches ein teures und schwierig zu handhabendes Reagenz ist. Die Anwendung dieser Synthesemethode ist bisher nur auf wenige Beispiele beschränkt.

Dagegen kann nach dem erfindungsgemäßen Verfahren wesentlich kostengünstiger gearbeitet werden. Es ist einfacher und auch ohne weiteres in technischem Maßstab durchführbar.

Als weitere Vorteile des erfindungsgemäßen Verfahrens sind beispielsweise die Möglichkeit der Durchführung bei Raumtemperatur und in wasserhaltigen Reaktionsmedien, die Möglichkeit anstelle von Natriumhydrid relativ billige Basen zu verwenden, die vergleichsweise einfache Aufarbeitung und die guten Ausbeuten zu nennen.

Das Verfahren 14 b zur Herstellung von Verbindungen der Formel (IX) wird im allgemeinen unter Verwendung von Verdünnungsmitteln durchgeführt. Als solche kommen praktisch alle inerten organischen Solventien in Frage. Hierzu gehören insbesondere aliphatische und

aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether wie Diethyl- und Dibutylether, Tetrahydrofuran und Dioxan. Alkohole wie Methanol, Ethanol, n- und iso-Propanol, n-, iso-, sek.- und tert.-Butanol, sek.- und tert.-Butanol sowie Dimethylsulfoxid.

Bei Arbeiten im Zweiphasenmedium werden neben 50 %iger wäßriger Natronlauge mit Wasser praktisch nicht mischbare Solventien wie z.B. Benzin, Benzol oder Toluol verwendet.

Als Basen können die bei Carbonyl-Olefinierungen üblichen Basen verwendet werden. Genannt seien Alkalihydroxide wie z.B. Natrium-, Kaliumhydroxid, Alkalialkoholate wie z.B. Natrium- und Kaliummethylat, -ethylat, -n- und -isopropylat, -n-, -iso-, sek.- und tert.-Butylat, Alkalihydride wie z.B. Natriumhydrid sowie Alkallithiumverbindungen wie z.B. Butyllithium.

Vorzugsweise werden als Basen Natrium- und Kaliumhydroxid sowie Natriummethylat und -ethylat verwendet.

Die Reaktionstemperaturen liegen im allgemeinen zwischen -70°C und +150°C, vorzugsweise zwischen -10 und +50°C. Die Umsetzung wird im allgemeinen bei Normaldruck durchgeführt.

Zur Durchführung des erfindungsgemäßen Verfahrens werden die Komponenten gewöhnlich in äquimolaren Mengen eingesetzt. Lediglich die Basen werden gewöhnlich in größerem Überschuß eingesetzt: bei Arbeiten im einphasigen System bis zu 30 Mol-%, vorzugsweise bis zu 15 Mol-%; bei Verwendung von 50 %iger Natronlauge als zweiter Phase im allgemeinen das 5 bis 15-fache der stöchiometrisch erforderlichen Menge.

Alkoholate werden gegebenenfalls in situ aus den Alkoholen und Alkalimetallen hergestellt.

Im allgemeinen wird die Base und gegebenenfalls der Katalysator in einem oder mehreren der genannten Verdünnungsmittel vorgelegt. Dazu werden, gegebenenfalls in einem der angegebenen Solventien gelöst, die Reaktionskomponenten - α-Chloro-benzylphosphonsäureester und Aldehyd - in der angegebenen Reihenfolge zugegeben. Zur Vervollständigung der Reaktion wird die Mischung unter Rühren mehrere Stunden im angegebenen Temperaturbereich gehalten.

Zur Aufarbeitung versetzt man die Reaktionsmischung mit Wasser, säuert gegebenenfalls mit Salzsäure an und extrahiert mit Methylenchlorid. Die organische Phase wird getrocknet und das Lösungsmittel wird im Vakuum abdestilliert. Das als Rückstand verbleibende Produkt wird gegebenenfalls durch Vakuumdestillation, oder falls es nicht unzersetzt destillierbar ist, durch sogenanntes "Andestillieren",

d.h. durch längeres Erhitzen unter vermindertem Druck auf mäßig erhöhte Temperaturen, gereinigt. Zur Charakterisierung dient der Siedepunkt oder der Brechungsindex.

Verwendet man bei Verfahren 14 c ß-(3,4-Dichlorphenyl)-ß-chlor-acrolein und Triphenylisopropylphosphoran als Ausgangsstoffe, so kann der Reaktionsverlauf durch folgendes Formelschema wiedergegeben werden:

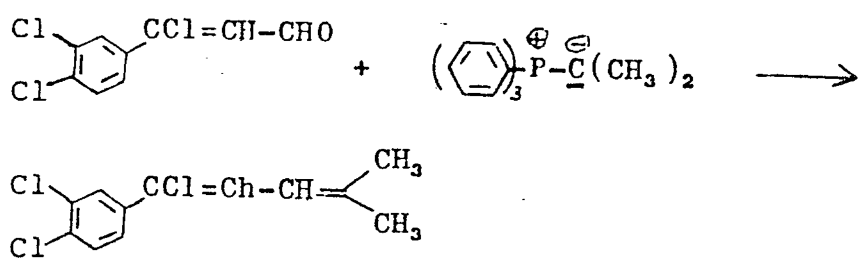

Die Herstellung des Phosphorans der Formel XVIII ist bekannt. Die Aldehyde der allgemeinen Formel XVII sind teilweise bekannt. Ihre Herstellung nach einem neuen Verfahren wird weiter unten beschrieben.

Die bei Verfahren 14 c verwendbaren Ausgangsstoffe sind durch die allgemeine Formel XVII definiert. Die bevorzugten und besonders bevorzugten Substituenten sind die gleichen wie bei Verfahren 1.

Als Beispiel für die Aldehyde der Formel XVII seien genannt: ß-Phenyl-ß-chlor-acrolein, ß-(4-Fluor-phe-

nyl)-ß-chlor-acrolein, ß-(4-Chlor-phenyl)-ß-chlor-acrolein, ß-(4-Brom-phenyl)-ß-chlor-acrolein, ß-(4-Methyl-phenyl)-ß-chlor-acrolein, ß-(Thiophen-2-yl)-ß-chlor-acrolein.

Verfahren 14 c ist eine Wittigreaktion und erfolgt nach im Prinzip bekannten Methoden.

Verwendet man bei Verfahren 14 d ß-(4-Methyl-phenyl)-ß-chloracrolein und Isopropylmagnesiumchlorid als Ausgangsstoffe, so kann der Reaktionsverlauf durch folgendes Formelschema wiedergegeben werden:

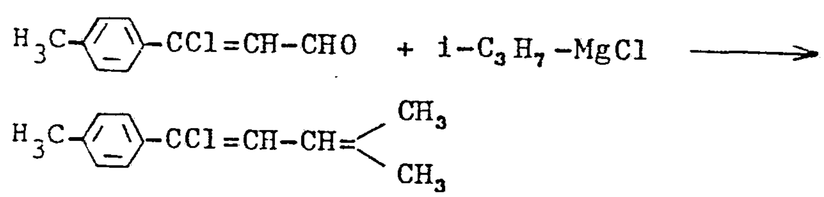

Die Herstellung von Isopropylmagnesiumchlorid XIX ist bekannt: Chem. Ber. 69 (1936), S. 1766.

Die Aldehyde der allgemeinen Formel XVII sind teilweise bekannt. Ihre Herstellung nach einem neuen Verfahren wird weiter unten beschrieben. Die bei Verfahren 14 d verwendeten Ausgangsstoffe sind durch die allgemeine Formel XVII definiert. Die bevorzugten und besonders bevorzugten Substituenten sind die gleichen wie bei Verfahren 1.

Als Beispiele für die Aldehyde der Formel XVII seien

genannt: ß-Phenyl-ß-chlor-acrolein, ß-(4-Fluorphenyl)-ß-chlor-acrolein, ß-(4-Chlor-phenyl)-ß-chloracrolein, ß-(4-Brom-phenyl)-ß-chlor-acrolein, ß-(4-Methylphenyl)-ß-chlor-acrolein, ß-(Thiophen-2-yl)-ß-chlor-acrolein.

Verfahren 14 d ist eine Grignard-Reaktion mit nachfolgender Dehydratisierung. Die Grignard-Reaktion erfolgt nach im Prinzip bekannter Weise. Es ist überraschend, daß aus dem intermediär gebildeten sekundären Alkohol verhältnismäßig leicht Wasser abgespalten werden kann.

Die Dehydratisierung erfolgt mit Säuren, vorzugsweise mit Schwefelsäure bei Temperaturen zwischen 0 und 50°C, vorzugsweise bei 20° bis 45°C. Die Isolierung der Diene erfolgt durch Eingießen in Wasser und Extrahieren in üblicher Weise.

Verwendet man bei Verfahren 15 (oben) 4-Fluor-acetophenon als Ausgangsstoff, so kann der Reaktionsverlauf durch folgendes Formelschema wiedergegeben werden:

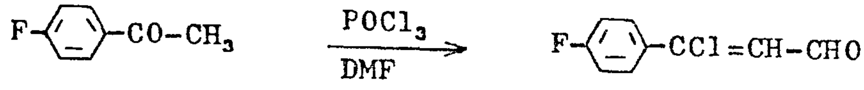


Die beim Verfahren 15 (oben) verwendbaren Ausgangsstoffe sind durch die allgemeine Formel (XVI)

$$Ar\text{-}CO\text{-}CH_3 \qquad (XVI)$$

definiert. Die bevorzugten und besonders bevorzugten Substituenten sind die gleichen wie bei Verfahren 1.

Als Beispiele für Acetophenone der Formel XVI seien genannt: Acetophenon, 4-Fluoracetophenon, 4-Chloracetophenon, 4-Brom-acetophenon, 4-Methyl-acetophenon, 2-Acetyl-thiophen, 4-Phenyl-acetophenon.

Es wurde gefunden, daß man nach dem erfindungsgemäßen Verfahren 15 die ß-Chloracroleine der allgemeinen Formel XVII in besonders einfacher Weise und mit besserer Ausbeute und Reinheit erhält, wenn man auf den in der Literatur (Proc. Chem. Soc. (London), 1958, 227; Angew. Chem. 71 ((1959), 573; Chem. Ber. 93 (1960), 2746; Chem. Ber. 98 (1965), 3554) empfohlenen Zusatz eines Verdünnungsmittels und die verlustreiche destillative Aufarbeitung verzichtet und die Substanz nach schonender Hydrolyse bei tiefer Temperatur kristallin isoliert.

Man geht beim erfindungsgemäßen Verfahren in der Weise vor, daß man das Acetophenon der allgemeinen Formel XVI in Dimethylformamid löst und bei 15 - 100°C, vorzugsweise bei 25 - 60°C tropfenweise mit Phosphoroxychlorid versetzt und noch bei der gleichen Temperatur 1 - 5 Stunden nachrührt.

Anschließend wird auf Eiswasser gegeben und mit Natronlauge ein pH-Wert von ca. 5 eingestellt. Die

Temperatur soll dabei nicht über 25°C steigen. Nach einigem Nachrühren wird abgesaugt, mit Wasser gewaschen und im Vakuum getrocknet.

Verwendet man bei Verfahren 16 (oben) Toluol und 4,4-Dimethyl-3-chlor-carbonylmethyl-γ-butyrolacton als Ausgangsstoffe, kann der Reaktionsablauf durch folgendes Formelschema wiedergegeben werden:

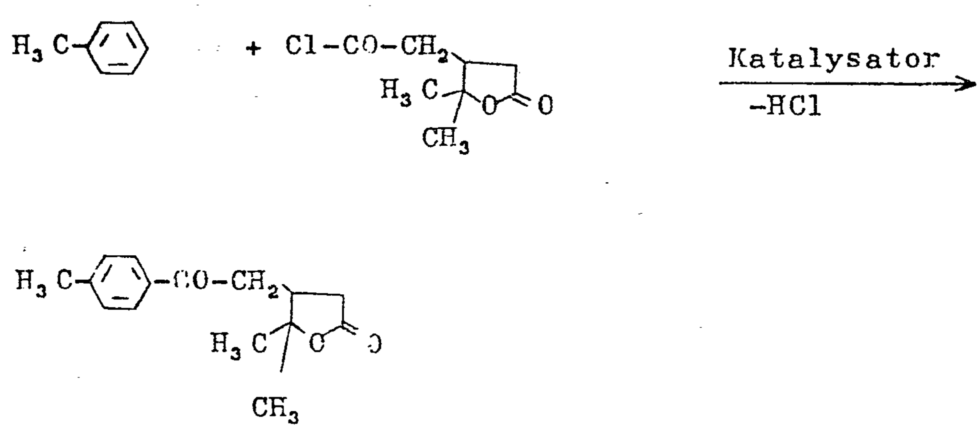


Die beim Verfahren 16 (oben) verwendbaren Ausgangsstoffe sind durch die Formeln XX und XXI definiert. Die Verbindung XXI ist neu; ihre Herstellung wird weiter unten beschrieben.

Die Verbindungen der Formel XX sind bekannt, beispielsweise seien genannt: Benzol, Fluorbenzol, Chlorbenzol,

Brombenzol, Toluol, Ethylbenzol, Thiophen, Furan, Diphenylether, o-Xylol, o-Dichlorbenzol. Als Katalysatoren kommen grundsätzlich alle üblichen Friedel-Crafts-Katalysatoren in Frage, wie Aluminiumchlorid, Zinntetrachlorid, Titantetrachlorid, Fluorwasserstoff, Bortrifluorid, Eisen(III)chlorid, Zinkchlorid, Polyphosphorsäuren, Perfluoralkansulfonsäure (gegebenenfalls in polymerer Form), sowie gegebenenfalls Mischungen derselben. Das Verfahren wird bevorzugt in Gegenwart eines Verdünnungsmittels durchgeführt. Es kommen in Frage: Methylenchlorid, Chloroform, Dichlorethan, Tetrachlorethan, Nitrobenzol, Nitromethan. Bevorzugt ist Methylenchlorid.

Die erfindungsgemäße Reaktion ist als äußerst überraschend zu bezeichnen, da erwartet werden mußte, daß auch der Lactonring in folgender Weise reagieren würde:

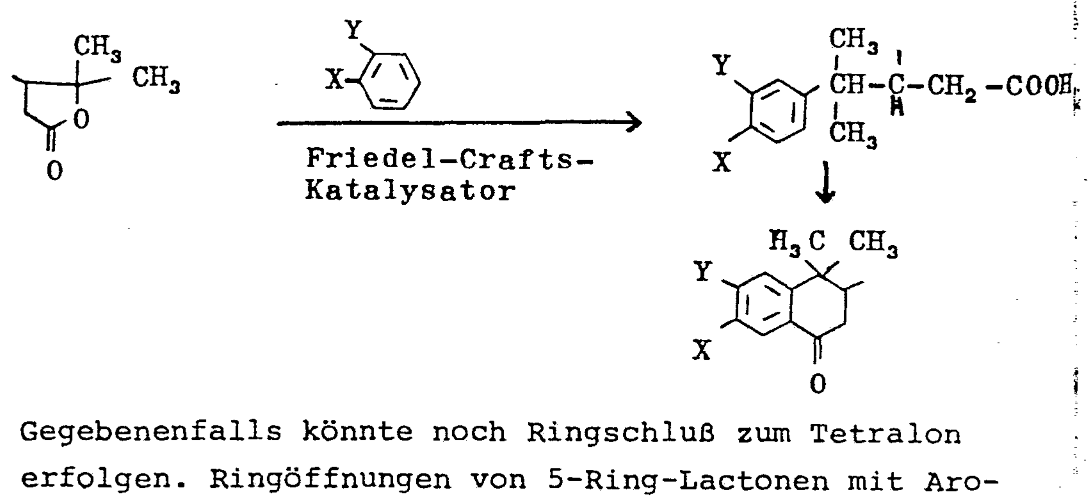


Gegebenenfalls könnte noch Ringschluß zum Tetralon erfolgen. Ringöffnungen von 5-Ring-Lactonen mit Aromaten nach Friedel-Crafts in der oben formulierten



BAD ORIGINAL

Weise sind bekannt und erfolgen unter sehr milden Bedingungen (Houben-Weyl; Band VI, 2 Seite 812 ff). Daß eine solche Reaktion des Lactons mit den Aromaten der Formel XXI nicht erfolgt, ist vor allem auch deshalb überraschend, da der Friedel-Crafts-Katalysator mindestens äquimolar, besser noch im Überschuß, eingesetzt werden muß.

Um Verfahren 16. (oben) im gewünschten Sinne ablaufen zu lassen,wird folgendermaßen vorgegangen:

Man legt das Säurechlorid XX in einem Verdünnungsmittel vor und fügt bei Temperaturen zwischen -10 und +5°C den Friedel-Crafts-Katalysator hinzu Anschließend tropft man den Aromaten, gegebenenfalls ebenfalls in einem Verdünnungsmittel gelöst, zu. Verwendet man sehr wirksame Friedel-Crafts-Katalysatoren, geschieht dies bei -10 bis +5°C (z.B. bei Aluminiumchlorid, Zinntetrachlorid), bei weniger wirksamen Friedel-Crafts-Katalysatoren (z.B. Zinkchlorid, Eisenchlorid, Titantetrachlorid, Perfluoralkansulfonsäuren) tropft man den Aromaten bei Raumtemperatur zu. Anschließend rührt man bei Raumtemperatur nach; bei weniger wirksamen Katalysatoren muß gegebenenfalls bei erhöhter Temperatur gearbeitet werden.

Verwendet man reaktionsträge Aromaten, wie beispielsweise Chlorbenzol, so empfiehlt es sich nicht, die Reaktion durch Temperaturerhöhung zu beschleunigen, sondern die Reaktionszeiten zu verlängern, um die unerwünschte Lactonringöffnung im oben erwähnten Sinn

zu verhindern. Bei stark desaktivierten Aromaten kann es notwendig sein, bei Temperaturen zwischen 30 und 80°C zu arbeiten, was sich jedoch auf die Ausbeuten negativ auswirkt.

Die Aufarbeitung erfolgt in üblicher Weise: die Lactone können durch Umkristallisieren gereinigt werden.

Verfahren 18 (oben) kann durch folgende Reaktionsgleichung beschrieben werden:

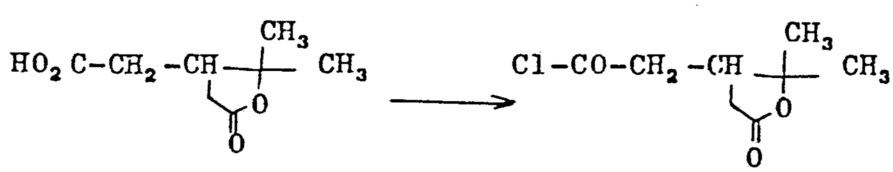

Die als Ausgangsstoff verwendete Säure der Formel XXII ist bekannt. Sie wird als "Terpenylsäure" bezeichnet und kann durch Oxidation von Terpentinöl erhalten werden. Das Säurechlorid der Formel XX ist neu. Daß eine glatte Umwandlung der Säure in das Säurechlorid gelingt, erscheint überraschend, da unter diesen Bedingungen üblicherweise auch eine Ringöffnung des Lactons erfolgt.

Verfahren 18. (oben) wird unter den Bedingungen durchgeführt, die zur Herstellung eines Säurechlorids aus einer Säure üblich sind. Bevorzugte Chlorierungsmittel sind Thionylchlorid und Phosgen. Es ist jedoch darauf zu achten, daß möglichst kurze Reaktionszeiten angewendet werden, um eine Nebenreaktion im oben erwähnten Sinne zu vermeiden. Die Aufarbeitung erfolgt in üblicher Weise. Das Säurechlorid kann durch Destillation gereinigt oder roh in Verfahren 16 (oben) eingesetzt werden.

Beispiel 1 (Verfahren 1)

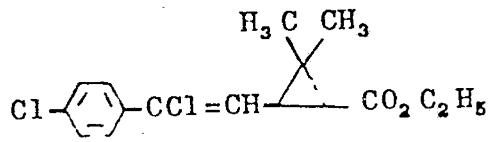

Man löst 35 g 5-(4'-Chlor)-phenyl-5-chlor-3-(1'-chlor-1'-methyl)-ethyl-pent-4-en-säureethylester in 200 ml Toluol und gibt 5 g Tetrabutylammoniumbromid zu. Anschließend wird auf ca. 30°C geheizt und innerhalb von 30 Minuten 56 g 50 %ige Kalilauge zugetropft. Die Temperatur wird dabei auf ca. 30 - 35°C gehalten. Anschließend rührt man noch 2 h bei 35°C nach, versetzt mit 200 g Eiswasser und trennt die organische Phase ab. Die Wasserphase wird noch zweimal mit je 50 ml Toluol ausgeschüttelt, die vereinigten organischen Phasen mit Wasser, dem etwas 1 n HCl zugesetzt wird, neutral gewaschen, mit $Na_2SO_4$ getrocknet und einrotiert. Durch Hochvakuumdestillation werden 26 g roher 2,2-Dimethyl-3-[2'-chlor-2'-(4'-chlor-phenyl)-vinyl]-cyclopropan-1-carbonsäureethylester erhalten (Isomerengemisch; eins der vier Isomeren entsteht ganz überwiegend), der durch eine zweite Destillation noch weiter gereinigt werden kann. Siedepunkt: 154 - 160°C/ 0,1 mbar.

Analog werden erhalten (Verfahren 1):

| Beispiel | Formel | Kp oder $n_D^{20}$ |
|---|---|---|
| 2 | 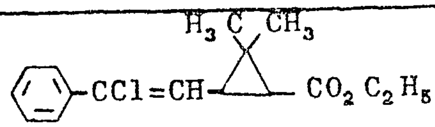 | 130-136° /0,2 mbar |
| 3 | 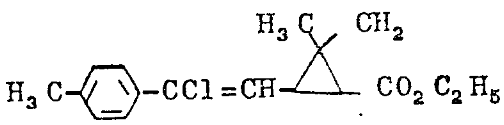 | 151-155° /0,2 mbar |
| 4 | 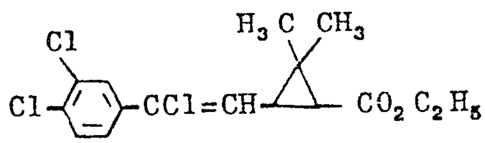 | 1.5644 |
| 5 | 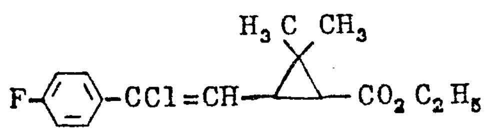 | 122-129° C/0,2 mbar |

Bespiel 6 (Verfahren 4)

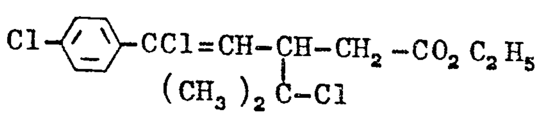

Man löst 142,5 g 4,4-Dimethyl-3-[2'-chlor-2'-(4'-chlorphenyl)-vinyl]-γ-butyrolacton in 500 ml Toluol. Dann gibt man 125 ml Thionylchlorid zu und erhitzt 6 h auf 80°C. Anschließend werden erneut 125 ml Thionylchlorid zugegeben und nochmals auf 80°C erhitzt. Anschließend wird das überschüssige Thionylchlorid und etwas Toluol unter Wasserausschluß bei Normaldruck abdestilliert (ca. 250 ml). Nach dem Abkühlen werden unter Kühlung innerhalb von 3 h 400 ml Ethanol, das mit Chlorwasserstoff gesättigt ist, bei 20°C zugetropft. Man rührt noch 3 h nach und läßt über Nacht stehen. Die Lösungsmittel werden unter vermindertem Druck (Wasserstrahlvakuum) abdestilliert. Der zurückbleibende rohe 5-(4'-Chlor-phenyl)-5-chlor-3-(1'-chlor-1'-methyl)-ethyl-pent-4-ensäureethylester wird direkt nach Verfahren 1 weiter umgesetzt.

Analog werden erhalten (Verfahren 4)

| Beispiel | Formel |
|---|---|
| 7 | 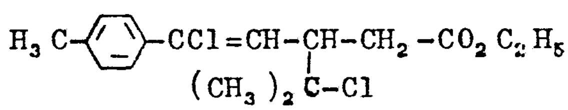 |
| 8 | 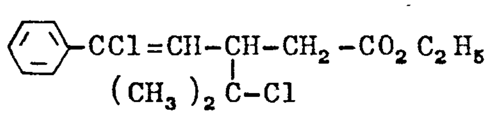 |
| 9 | 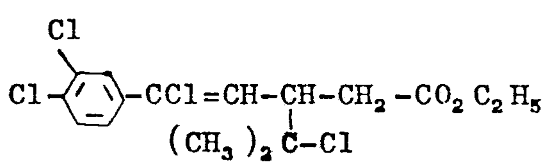 |

Beispiel 10 (Verfahren 4)

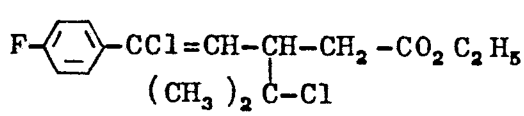

Man mischt in einem 0,7 l-Autoklaven 134 g 4,4-Dimethyl-3-/2'-chlor-2'-(4'-fluorphenyl)-vinyl7-γ-butyrolacton mit 120 g Thionylchlorid. Anschließend werden 50 g Ethanol zugepumpt. Dann heizt man den Autoklaven noch 1 h auf 80°C, läßt abkühlen und entspannt. Überschüssiges Thionylchlorid bzw. Schwefelsäureethylester werden durch Destillation im Wasserstrahlvakuum entfernt. Der Rückstand besteht im wesentlichen aus 5-(4'-Fluor)-phenyl-5-chlor-3-(1'-chlor-1'-methyl)-ethyl-pent-4-en-säureethylester und wird direkt nach Verfahren 1 weiter umgesetzt.

Beispiel 11 (Verfahren 6 a)

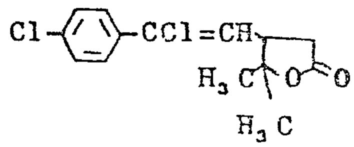

Man löst 55,9 g 4,4-Dimethyl-3-(4'-chlor)-phenylacyl-γ-butyrolacton in 300 ml Toluol, dosiert 87 g Phosphorpentachlorid hinzu und rührt bei Raumtemperatur solange nach, bis alles in Lösung gegangen ist (ca. 8 h). Dann tropft man bei 20 - 50°C 500 ml Wasser hinzu und rührt 4 h nach. Anschließend wird die Toluolphase abgetrennt, mit Natriumsulfat getrocknet und im Wasserstrahlvakuum eingeengt. Es bleibt 4,4'-Dimethyl-3-/2'-chlor-2'-(4'-chlor-phenyl)-vinyl7-γ-butyrolacton (E- und Z-Isomeres) zurück.

Analog Verfahren 6 a werden erhalten:

| Beispiel | Formel |
|---|---|
| 12 | 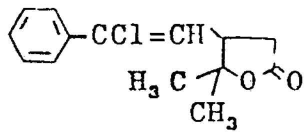 |
| 13 | 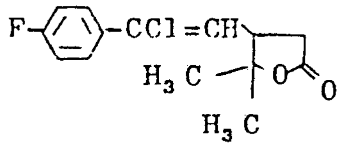 |
| 14 | 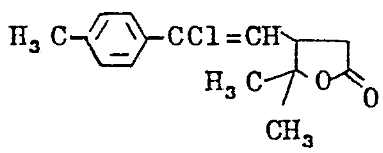 |
| 15 | 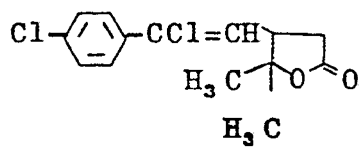 |

**Beispiel 16** (Verfahren 6 b)

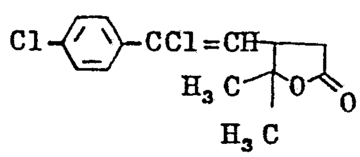

42,8 g rohes 4,4-Dimethyl-3-[2'-chlor-2'-(4'-chlorphenyl)-vinyl]-2-ethoxycarbonyl-γ-butyrolacton werden in 400 ml 25 %iger Schwefelsäure suspendiert und bei Normaldruck so lange Wasser und Ethanol abdestil

liert, bis die Innentemperatur 115 - 120°C erreicht hat. Dan erhitzt man noch 10 h bei dieser Temperatur (ohne weiteres Abdestillieren), läßt abkühlen und extrahiert mit Methylenchlorid. Nach Trocknen mit Natriumsulfat und Abziehen des Lösungsmittels hinterbleibt ein dunkler Rückstand, der nach kurzem Stehen teilweise kristallisiert. Es handelt sich aufgrund des NMR-Spektrums um 4,4-Dimethyl-3-[2'-chlor-2'-(4'-chlor-phenyl)-vinyl]-γ-butyrolacton (E- und Z-Isomeres).

$^{1}H$-NMR ($CDCl_3$): δ (ppm) = 1,3 (3 H, s), 1.45 (3 H, s); 2,9 - 3,7 (2 H + 1 H, m); 5,8 - 6,1 (1 H, m); 7,3 - 8,1 (4 H, m).

<u>Beispiel 17</u> (Verfahren 9)

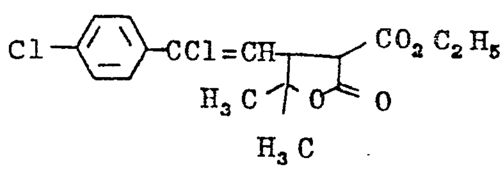

Man löst 3,5 g Natrium in 350 ml Ethanol und tropft bei Raumtemperatur 24 g Malonsäurediethylester hinzu. Dann werden bei 30 - 35°C 36,4 g 2,2-Dimethyl-3-[2'-chlor-2'-(4'-chlorphenyl)-vinyl]-oxiran langsam zugetropft. Nach beendetem Zutropfen hält man noch 4 h auf 35°C und läßt dann abkühlen. (An dieser Stelle kann sich gegebenenfalls direkt Verfahren 6 b anschließen).

Man destilliert im Vakuum ein Teil des Ethanols ab, versetzt mit Eiswasser und stellt sauer. Dann wird mit Methylenchlorid extrahiert, die organische Phase mit Natriumsulfat getrocknet und das Lösungsmittel abdestilliert. Der Rückstand wiegt 42,8 g und besteht - wie durch IR-Spektrum, NMR-Spektrum und Massenspektrum belegt -hauptsächlich aus 4,4-Dimethyl-3-/2'-chlor-2'-(4'-chlorphenyl)-vinyl7-2-ethoxycarbonyl-γ-butyrolacton. Brechungsindex: $n_D^{20}$: 1,535.

Beispiel 18 (Verfahren 12)

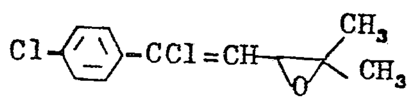

91 g (0,4 Mol) 1-(4'-Chlor-phenyl)-1-chlor-4,4-dimethyl-1,3-butadien werden in 640 ml Methylenchlorid gelöst und 160 g pulverisiertes wasserfreies Natriumcarbonat zugegeben. Dann dosiert man bei Raumtemperatur 69 g (0,4 Mol) m-Chlor-perbenzoesäure hinzu und rührt 2 h bei Raumtemperatur nach. Anschließend wird abgesaugt, mit $CH_2Cl_2$ nachgewaschen und die Methylenchloridlösung zweimal mit viel Natriumbicarbonatlösung geschüttelt. Nach Neutralwaschen mit Wasser, wird die Methylenchloridphase mit $Na_2SO_4$ getrocknet und einrotiert. Der hellgelbe flüssige Rückstand ($n_D^{20}$ = 1,563) enthält mach GC drei Komponenten mit der Masse m/e = 242 (GC/MS). Der Gehalt an den gewünschten stereoisomeren Epoxiden beträgt 88,3 %. Das Produkt (83 g) wird direkt in die nächste Stufe eingesetzt.

$^{1}H$-NMR ($CDCl_3$): $\delta$ (ppm) = 1,3 - 1,45 (6 H, m), 3,1 - 3,25 (1 H, m), 5,8 - 6,05 (1 H, dd), 7,3 - 7,65 (4 H, m).

Beispiel 19 (Verfahren 14 a)

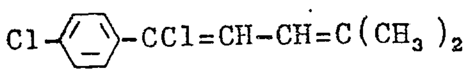

Man löst 17 g Kaliumhydroxid in 125 ml Wasser und 125 ml Methanol und gibt 155 g 4-Chloracetophenon hinzu. Bei 50°C werden 80 g Isobutyraldehyd zugetropft (innerhalb von 1,5 Stunden). Nach 3,5 Stunden wird auf Raumtemperatur gekühlt und mit Essigsäure neutralisiert (ca. 20 ml). Das dimere Kondensationsprodukt wird abgesaugt, mit Methanol gewaschen und an der Luft getrocknet. Ausbeute 185 g (88,5 % d.Th.) Smp. 140°C. Der Feststoff wird mit 5 g Natriumacetat vermischt und im Wasserstrahlvakuum destilliert. Man erhält 175 g eines bei Raumtemperatur erstarrenden gelben Öls, das ein Gemisch aus 2-Methyl-5-oxo-5-(4'-chlor-phenyl)-2-penten und 2-Methyl-5-oxo-5-(4'-chlor-phenyl)-3-penten darstellt. Das erstere ist ein Feststoff und kristallisiert bevorzugt aus, Siedepunkt des Isomerengemisches: Kp = 155 - 157°C/18 mbar.

Man löst 104,25 g (0,5 Mol) des oben erhaltenen Isomerengemisches in 500 ml Toluol und dosiert 104 g Phosphorpentachlorid bei 0°C langsam hinzu. Man rührt solange bei 0 - 10°C, bis alles $PCl_5$ in Lösung gegangen ist, gibt 5 g Tetrabutylammoniumbromid zu und tropft unter guter Kühlung bei 0 - 10°C 224 g (2 Mol) 50 %ige

Kalilauge zu. Anschließend trennt man die Toluolphase sofort ab, wäscht neutral und rotiert ein. Anschließend wird das rohe Dien überdestilliert (Kp = 130 - 159 $^{O}$C/0,2 mm) und anschließend durch eine zweite Destillation gereinigt. Man erhält 67 g 1-(4'-Chlor-phenyl)-1-chlor-4,4-dimethyl-1,3-butadien vom Siedepunkt 100 - 109 $^{O}$C/0,1 mm.

<u>Beispiel 20</u> (Verfahren 14 a)

Analog wird aus 2-Methyl-5-oxo-5-phenyl-2(3)-penten (hergestellt nach J. Org. Chem. <u>25</u>, S. 272) 1-Phenyl-1-chlor-4,4-dimethyl-1,3-butadien erhalten. Kp = 92 - 96 $^{O}$C/0,08 mm.

<u>Beispiel 21</u> (Verfahren 14 b)

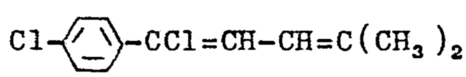

4,6 g (0,2 Mol) Natrium werden portionsweise in 100 ml trockenem Ethanol gelöst. Wenn sich alles Natrium aufgelöst hat, werden 100 ml wasserfreies Tetrahydrofuran zugesetzt und bei 0 $^{O}$C unter Rühren 59,4 g (0,2 Mol) 4-Chlor-α-chlor-benzyl-phosphonsäurediethylester, gelöst in 50 ml wasserfreiem THF, zugetropft. Nachdem noch 1 Stunde bei 0 - 10 $^{O}$C nachgerührt wurde, werden 16,8 g (0,2 Mol) ß,ß-Dimethylacrolein, gelöst in 30 ml wasserfreiem Tetrahydrofuran, unter Rühren zugetropft. Anschließend wird noch 12 Stunden bei Raumtemperatur nachgerührt. Die Reaktionsmischung wird dann mit 600 ml Wasser versetzt und zweimal mit je 300 ml Methylenchlorid extrahiert. Die organische Phase wird abgetrennt, über Magnesiumsulfat getrocknet, das Lösungsmittel im Wasserstrahlvakuum abdestilliert und der Rückstand im Vakuum destilliert. Man erhält 25,2 g 1-(4'-chlor-phenyl)-1-chlor-4,4-dimethyl-1,3-butadien (Isomerengemisch) als gelbes, nach einiger Zeit teilweise kristallisierendes Öl, mit dem Siedepunkt 125 - 134°C/2 Torr.

Analog erhält man (Verfahren 14 b)

<u>Beispiel 22</u>

1-(4'-Methoxy-phenyl)-1-chlor-4,4-dimethyl-1,3-butadien

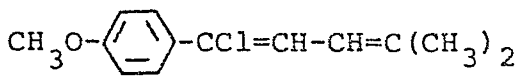

Kp = 130 - 145°C/ 2 Torr

<u>Beispiel 23</u>

1-(Phenyl)-1-chlor-4,4-dimethyl-1,3-butadien

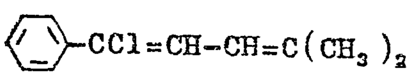

Kp = 120 - 135°C/2 Torr

Beispiel 24

1- (4'-Fluor-phenyl)-1-chlor-4,4-dimethyl-1,3-butadien

Beispiel 25

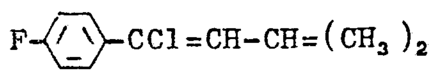

1-(4'-Methyl-phenyl)-1-chlor-4,4-dimethyl-1,3-butadien

Beispiel 26

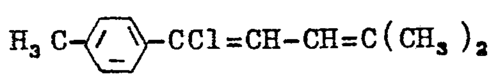

Beispiel 27

1-(3,4'-Methylendioxy-phenyl)-1-chlor-4,4-dimethyl-1,3-butadien.

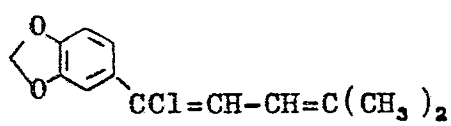

Die als Vorprodukte benötigten α-Hydroxy-benzyl-phosphonsäureester der Formel können wie folgt hergestellt werden:

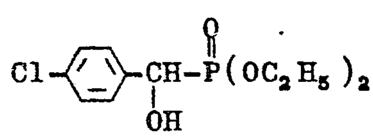

Beispiel 28

In ein Gemisch aus 20,7 g (0,15 Mol) Diethylphosphit und 1,09 g (0,0109 Mol) Triethylamin werden innerhalb einer Stunde unter Wasserkühlung bei 50 - 70°C 21 g (0,150 Mol) 4-Chlorbenzaldehyd eindosiert. Der Reaktionsansatz wird 1 Stunde bei 70°C nachgerührt. Nach dem Abkühlen wird der Ansatz mit 40 g Toluol aufgenommen und mehrfach mit verdünnter Salzsäure und kaltem Wasser nachgewaschen. Die organische Schicht wird abgetrennt und im Vakuum vom Lösungsmittel befreit. Der feste Rückstand schmilzt bei 70 - 72°C. Die Ausbeute beträgt 37 g (88,5 % der Theorie) an 4-Chloro-α-hydroxy-benzyl-phosphonsäure-diethylester.

Beispiel 29

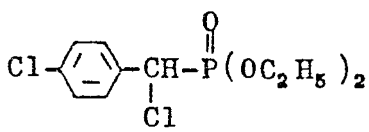

Einem Gemisch aus 20,2 g (0,0725 Mol) 4-Chloro-α-hydroxy-benzyl-phosphonsäure-diethylester, 65 g Dichlormethan und 5,8 g (0,0725 Mol) Pyridin werden bei 20 - 40°C unter leichter Wasserkühlung innerhalb von etwa 1 Stunde 9,2 g (0,0768 Mol) Thionylchlorid hinzugesetzt. Das Reaktionsgemisch wird dann 3 Stunden unter Rückfluß erhitzt und 12 Stunden ohne weitere Wärmeeinwirkung nachgerührt. Das Gemisch wird auf etwa 100 g Eiswasser gegossen, die organische Phase abgetrennt und getrocknet. Nach Abdestillieren des Lösungsmittels wird der Rückstand bei 6 Torr und 45 °C eingeengt. Man erhält 21 g (97,7 % der Theorie) 4-Chloro-α-chloro-benzyl-phosphonsäure-diethylester als gelbes viskoses Öl mit einer Reinheit von 98,6 % (Gaschromatogramm) und einem Brechungsindex von $n_D^{24}$: 1.5250.

Analog werden erhalten:

| Beispiel | Formel | |
|---|---|---|
| 30 | 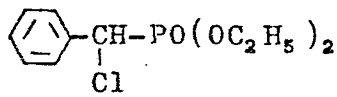 | $n_D^{23}$ = 1.5117 |
| 31 | 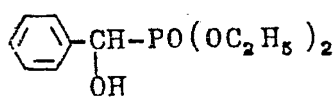 | Smp. 75° C |
| 32 | 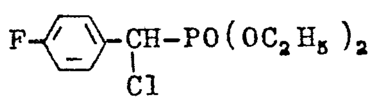 | |
| 33 | 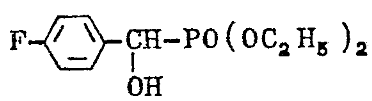 | Smp. 26° C |

**Beispiel 34** (Verfahren 14 c)

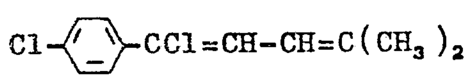

Zu einer Suspension von 22,7 g (0,0525 Mol) Triphenylisopropyl-phosphoniumjodid in 100 ml Tetrahydrofuran werden bei 0°C 0,055 Mol n-Butyl-lithium in 30 ml Hexan

getropft. Es wird 1 Stunde bei 0°C nachgerührt. Dann wird in einem Guß eine Lösung von 10,1 g (0,05 Mol) ß-(4-Chlor-phenyl)-ß-chlor-acrolein in 100 ml THF zugegeben und dann 10 Stunden bei 20°C nachgerührt. Das Reaktionsgemisch wird darauf eingeengt, mit 100 ml Wasser versetzt und zweimal mit je 50 ml Ether extrahiert. Die vereinigten Etherextrakte werden über $Na_2SO_4$ getrocknet und eingeengt. Es verbleibt ein Rückstand, der bei Zugabe von ca. 50 ml Petrolether kristallisiert. Die Kristalle werden abgesaugt und noch einmal aus Petrolether umkristallisiert. Man erhält 8 g (70,5 % d.Th.) 1-(4'-Chlor-phenyl)-1-chlor-4,4-dimethyl-1,3-butadien in Form von hellgelben Kristallen. Smp. 53°C.

<u>Beispiel 35</u> (Verfahren 14 d)

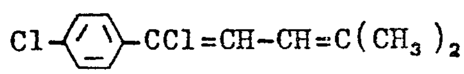

Zu einer Lösung von 0,4 Mol Isopropylmagnesiumchlorid in 40 ml Isopropylchlorid (hergestellt nach J. Houben et. al. Chem. Ber. <u>69</u> (1936), 1766) werden bei 20°C 40 g (0,2 Mol) ß-Chlor-ß-(4-chlorphenyl)-acrolein in 200 ml Tetrahydrofuran getropft. Es wird 12 Stunden nachgerührt. Dann wird die Reaktionsmischung unter Rühren in eine Mischung von 500 g Eis und 100 ml $H_2SO_4$ gegossen. Es wird zweimal mit je 100 ml Ether extrahiert. Die vereinigten Etherextrakte werden über Natriumsulfat getrocknet und bei 50°C am Rotationsverdampfer eingeengt.

Der Rückstand wird in 50 ml Eisessig aufgenommen. Zu dieser Lösung werden 2 ml konz. $H_2SO_4$ in der Weise getropft, daß die Temperatur 45°C nicht übersteigt. Es wird 4 h bei 20°C nachgerührt. Dann wird die Reaktionsmischung in Wasser gegeben. Es wird zweimal mit je 100 ml Ether extrahiert. Die vereinigten Etherextrakte werden über $Na_2SO_4$ getrocknet, eingeengt und destilliert. Man erhält 23 g 1-(4'-Chlorphenyl)-1-chlor-4,4-dimethyl-1,3-butadien in Form eines gelben Öles, das beim Abkühlen teilweise kristallisiert. Kp. = 140 - 145°C/4 mm.

Beispiel 36 (Verfahren 15)

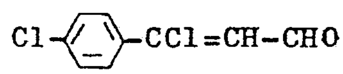

772,5 g (5 Mol) 4-Chloracetophenon werden in 2,5 l Dimethylformamid gelöst, bei 40 - 45°C unter leichter Kühlung innerhalb von 2 Stunden tropfenweise mit 1570 g Phosphoroxichlorid versetzt, 1 Stunde verrührt, auf 25 l Eiswasser ausgetragen und durch Zutropfen von ca. 2,1 l konzentrierter Natronlauge ein pH-Wert von 5 eingestellt. Die Temperatur wird während der Neutralisation durch Einwerfen von Eis unter 25°C gehalten. Anschließend wird noch 1 Stunde bei pH 5 und bei einer Temperatur unter 25°C nachgerührt. Der helle kristalline Niederschlag wird abgesaugt, gründlich mit Wasser gewaschen und bei 50°C im Vakuum getrocknet. Ausbeute 740 g (= 74 % d. Th.) Smp. 98°C.

Beispiel 37 (Verfahren 16)

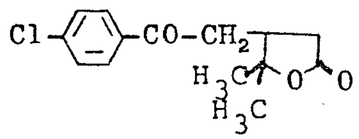

Man legt 80 g Aluminiumchlorid in 300 ml Methylenchlorid vor und tropft 59 g 4,4-Dimethyl-3-chlorcarbonylmethyl-γ-butyrolacton in 150 ml Methylenchlorid, gelöst bei 0 - 5°C, zu. Anschließend tropft man 32,2 g Chlorbenzol in 50 ml Methylenchlorid gelöst ebenfalls bei 0 - 5°C zu. Dann läßt man auf Raumtemperatur kommen und rührt noch 7 h bei Raumtemperatur nach. Nach Eingießen des Ansatzes in Eiswasser wird die organische Phase abgetrennt und neutral gewaschen. Nach Trocknen und Abdestillieren des Lösungsmittels erhält man 76 g rohes 4,4-Dimethyl-3-(4'-chlor-phenacyl)-γ-butyrolacton, das aus Ethanol umkristallisiert wird. Smp. 104°C.

Beispiel 38 (Verfahren 16)

Man legt 5,3 g Aluminiumchlorid in 20 ml Methylenchlorid vor und tropft 3,5 g 4,4-Dimethyl-3-chlorcarbonylmethyl-γ-butyrolacton in 10 ml Methylenchlorid gelöst bei 0 - 5°C zu. Anschließend tropft man 2 g Toluol in 5 ml Methylenchlorid ebenfalls bei 0 - 5°C zu. Dann läßt man auf Raumtemperatur kommen und rührt noch 3 Stunden bei Raumtemperatur nach. Nach Eingießen des Ansatzes in 100 ml Eiswasser wird die organische Phase abgetrennt und mit Wasser neutral gewaschen. Nach Trocknen mit Natriumsulfat und Abestillieren des Lösungsmittels wird der Rückstand aus wenig Toluol umkristallisiert. Man erhält 4,1 g 4,4-Dimethyl-3-(4'-methyl -phenacyl)-γ-butyrolacton vom Smp. 117°C.

Beispiel 39 (Verfahren 16)

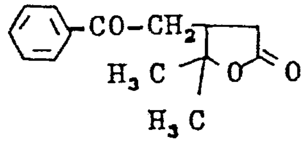

Man legt 132,5 g Aluminiumchlorid in 400 ml Methylenchlorid vor und tropft 95,2 g 4,4-Dimethyl-3-chlorcarbonylmethyl-γ-butyrolacton in 100 ml Methylenchlorid gelöst bei -5 bis 0°C zu. Anschließend tropft man 43 g Benzol in 50 ml Methylenchlorid bei 0 - 10°C zu. Dann läßt man auf Raumtemperatur kommen und rührt noch 4 h nach. Nach Eingießen des Ansatzes in 1 Liter Eiswasser wird die organische Phase abgetrennt und neutral gewaschen. Nach Trocknen und Abdestillieren des Lösungsmittels erhält man 115 g Rohprodukt, das nach Umkristallisieren aus Ethanol bei 96°C schmilzt. Es handelt sich lt. Kernresonanzspektrum und Massenspektrum um 4,4-Dimethyl-3-phenacyl-γ-butyrolacton.

Analog Beispiel 37 werden erhalten (Verfahren 16)

| Beispiel | Formel | Fp. |
| --- | --- | --- |
| 40 | 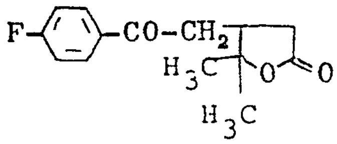 | 113°C(Ethanol) |
| 41 | 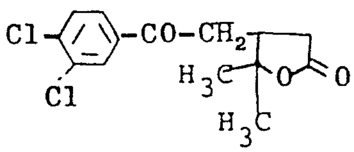 | 143°C |

**Beispiel 42**

Herstellung von 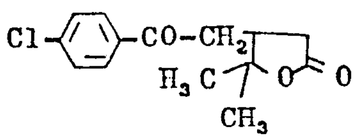

analog J.Org.Chem. 39 (1974), 2601

Man mischt 108 g Kaliumcarbonat, gelöst in 386 ml Wasser, mit 118,8 g 4-Chlorbenzoylessigsäureethylester und 68 g α-Chlorisobutyraldehyd. Man rührt 3 Tage bei Raumtemperatur, 7 h bei 50°C und 1 h bei 70°C, gibt dann auf 2 l Wasser und filtriert ab. Nach Waschen mit Petrolether wird an der Luft getrocknet. Ausbeute: 124 g vom Smp. 142°C.

100 g des Feststoffs werden mit 700 g Natronlauge,hergestellt aus 200 g NaOH und 500 g Wasser, 15 h auf 60°C erhitzt. Nach dem Abkühlen wird mit 10 %iger Salzsäure sauer gestellt und die ausgefallene p-Chlorbenzoesäure abgesaugt. Die Mutterlauge wird mit Methylenchlorid extrahiert. Die Methylenchloridphase wird zweimal mit Bicarbonatlösung und dann mit Wasser gewaschen, getrocknet und einrotiert. Der Rückstand kristallisiert nach einigem Stehen. Nach zweimaligem Umkristallisieren aus Ethanol erhält man 28,7 g schwach gelbliche Kristalle vom Smp. 103 - 104°C. Sie bestehen aus 4,4-Dimethyl-3-(4'-chlor -phenacyl)-γ-butyrolacton.

Beispiel 43 (Verfahren 18)

Man vermischt 172 g 4,4-Dimethyl-3-carboxymethyl-γ-butyrolacton mit 600 ml Thionylchlorid und erhitzt 1 h auf 80°C. Dann destilliert man das überschüssige Thionylchlorid bei Normaldruck ab, die letzten Reste im Wasserstrahlvakuum. Der Rückstand besteht aus 4,4-Dimethyl-3-chlorcarbonylmethyl-γ-butyrolacton ($n_D^{20}$ = 1,484) und kann direkt für Verfahren 16 verwendet werden. Es kann jedoch auch noch durch Destillation weiter gereinigt werden: Siedepunkt: 130 - 140°C/0,3 mbar (bei größeren Mengen muß am Dünnschichter destilliert werden). Das reine Säurechlorid ist fest. Smp. 64°C.

atentansprüche

. Verfahren zur Herstellung von 3-(Arylvinyl)-2,2-dimethyl-cyclopropan-1-carbonsäureester der allgemeinen Formel (I)

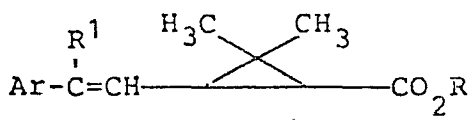

(I)

in welcher

Ar für substituierte oder unsubstituierte carbocyclische oder heterocyclische aromatische Reste steht und

R für Alkyl oder für einen bei Pyrethroiden üblichen Alkoholrest steht, und

$R^1$ für Wasserstoff, Fluor oder Chlor steht,

dadurch gekennzeichnet, daß man Verbindungen der allgemeinen Formel (II)

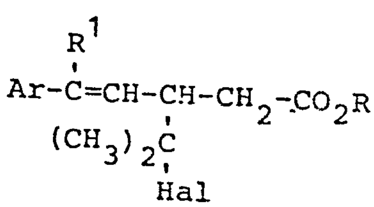

(II)

in welcher

Ar, $R^1$ und R die oben angegebene Bedeutung besitzen und

Hal für Chlor oder Brom steht,

mit einer gegebenenfalls wäßrigen Base gegebenenfalls in Gegenwart eines Verdünnungsmittels in Gegenwart eines Phasentransferkatalysators umsetzt.

2. Verfahren gemäß Anspruch 1, dadurch gekennzeichnet, daß man nicht zu starke Basen verwendet und in Gegenwart eines Phasentransferkatalysators arbeitet.

3. Verbindungen der allgemeinen Formel (II), in welcher Ar, $R^1$, R und Hal die unter Anspruch 1 (oben) angebebene Bedeutung besitzen.

4. Verfahren zur Herstellung der Verbindungen der allgemeinen Formel (II), dadurch gekennzeichnet, daß Verbindungen der allgemeinen Formel (III)

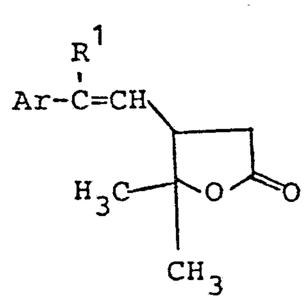

(III)

in welcher

Ar und $R^1$ die unter 1. (oben) angegebene Bedeutung haben,

mit einem Halogenierungsmittel sowie gleichzeitig oder nacheinander mit einem Alkohol der Formel (IV)

R-OH (IV)

in welcher

R die unter 1. (oben) angegebene Bedeutung besitzt,

gegebenenfalls in Gegenwart eines Verdünnungsmittels und gegebenenfalls in Gegenwart von gasförmigem Halogenwasserstoff, umgesetzt werden.

Verbindungen der Formel (III), in welcher Ar und $R^1$ die in Anspruch 1 (oben) angegebene Bedeutung haben.

Verfahren zur Herstellung der neuen Verbindungen der Formel (III), dadurch gekennzeichnet, daß man

a) Verbindungen der Formel (V)

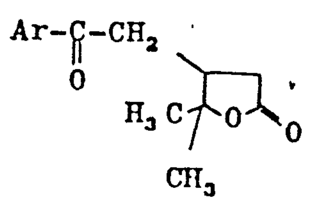

(V)

in welcher
Ar die oben angegebene Bedeutung hat,

mit Phosphorpentachlorid umsetzt oder

b) Verbindungen der Formel (VI)

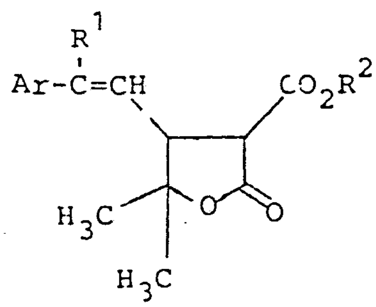

(VI)

in welcher
Ar und $R^1$ die unter 1. (oben) angegebene Bedeutung haben und
$R^2$ $C_{1-4}$-Alkyl bedeutet,

decarbalkoxyliert oder verseift und decarboxyliert oder

c) Verbindungen der Formel (V)

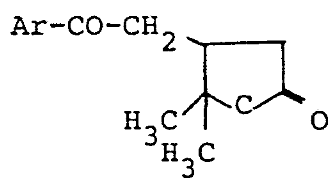

(V)

in welcher
Ar die oben angegebene Bedeutung hat,

reduziert und Wasser abspaltet.

. Verbindungen der Formel (V), in welcher Ar die in Anspruch 1. (oben) angegebene Bedeutung hat, jedoch nicht unsubstituiertes Phenyl bedeutet.

. Verbindungen der Formel (VI), in der Ar, $R^1$ und $R^2$ die in Anspruch 6. (oben) angegebene Bedeutung haben.

. Verfahren zur Herstellung von Verbindungen der allgemeinen Formel (VI), dadurch gekennzeichnet, daß man Verbindungen der allgemeinen Formel (VII)

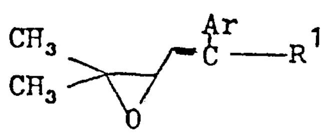

(VII)

in welcher

Ar und $R^1$ die unter 1. (oben) angegebene Bedeutung haben

mit Malonestern der Formel (VIII)

$$CH_2(CO_2R^2)_2 \qquad (VIII)$$

in welcher

$R^2$ $C_{1-4}$-Alkyl bedeutet,

in Gegenwart einer Base sowie gegebenenfalls in Gegenwart eines Verdünnungsmittels umsetzt.

10. Verfahren gemäß Anspruch 9, dadurch gekennzeichnet, daß man unterhalb von 50°C arbeitet.

11. Verbindungen der Formel (VII), in welcher Ar und $R^1$ die in Anspruch 1. (oben) angegebene Bedeutung haben.

12. Verfahren zur Herstellung der Verbindungen der Formel (VII), dadurch gekennzeichnet, daß man Verbindungen der Formel (IX)

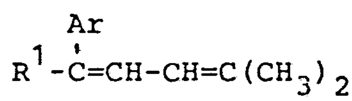

(IX)

.n welcher

Ar und F$^1$ die oben ancegebene Bedeutung haben,

oxidiert.

13. Verbindungen der Formel (IX), in welcher Ar und $R^1$ die in Anspruch 1. (oben) angegebene Bedeutung haben.

14. Verfahren zur Herstellung der Verbindungen IX, dadurch gekennzeichnet, daß man

a) Verbindungen der Formel X A oder X B

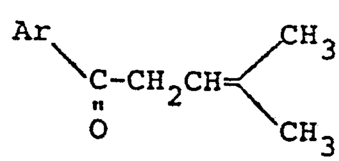

(X A)

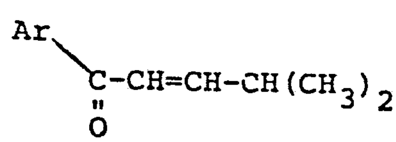

(X B)

in welcher

Ar die unter 1. (oben) angegebene Bedeutung besitzt,

oder ein Gemisch derselben, mit Phosphorpentachlorid und anschließend einer Base, umsetzt oder

b) Verbindungen der Formel (XI)

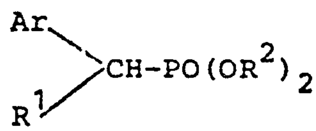

(XI)

in welcher

Ar, $R^1$ und $R^2$ die unter 6. (oben) angegebene Bedeutung haben,

mit Dimethylacrolein der Formel (XII)

$(CH_3)_2C=CH-CHO$ (XII)

umsetzt oder

c) Verbindungen der Formel (XVII)

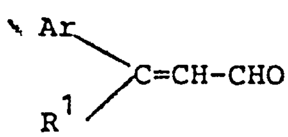

(XVII)

in welcher

Ar und $R^1$ die unter 1. (oben) angegebene Bedeutung haben,

mit der Verbindung der Formel (XVIII)

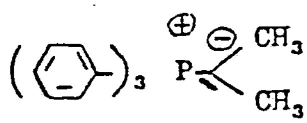 (XVIII)

umsetzt

oder

d) Verbindungen der Formel XVII mit Isopropylmagnesiu chlorid der Formel (XIX)

$$(i\text{-}C_3H_7\text{-}MgCl) \qquad (XIX)$$

umsetzt.

15. Verfahren zur Herstellung der Verbindungen der allgemeinen Formel (XVII)

$$Ar-\overset{R^1}{\overset{|}{C}}=CH-CHO$$

in welcher

Ar und $R^1$ die in Anspruch 1. (oben) angegebene Bedeutung haben,

dadurch gekennzeichnet, daß man Acetophenone der allgemeinen Formel (XVI)

$$Ar\text{-}CO\text{-}CH_3 \qquad (XVI)$$

in welcher

Ar die in Anspruch 1. (oben) angegebene Bedeutung hat, ohne Zusatz eines Verdünnungsmittels mit $POCl_3$ und Dimethylformamid umsetzt und die Reaktionsprodukte nach Hydrolyse ohne destillative Aufarbeitung isoliert.

6. Verfahren zur Herstellung von Verbindungen der Formel (V), die sich unter der Formel (V A) zusammenfassen lassen

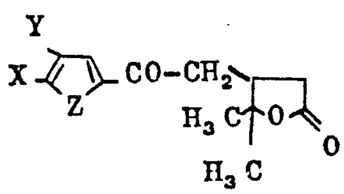

(V A)

in welcher

X für Wasserstoff, $C_{1-4}$-Alkyl, Halogen, Aryl, Aralkyl, Aryloxy, Arylthio, $C_{1-4}$-Alkoxy oder $C_{1-4}$-Alkylthio steht,

Y für Wasserstoff steht oder gleich X ist, oder

X und Y für Methylendioxy oder Ethylendioxy stehen und

Z für O, S oder -CH=CH- steht,

gefunden, das dadurch gekennzeichnet ist, daß man die Verbindung der Formel (XX)

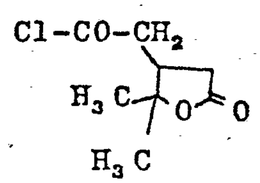

(XX)

mit Verbindungen der Formel (XXI)

in welcher

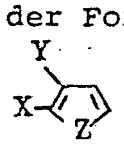

(XXI)

X, Y und Z die in Anspruch 16. angegebene Bedeutung haben,

in Gegenwart eines Friedel-Crafts-Katalysators und gegebenenfalls in Gegenwart eines Verdünnungsmittels umsetzt.

17. Verbindung der Formel (XX).

18. Verfahren zur Herstellung der Verbindung (XX), dadurch gekennzeichnet, daß die Verbindung der Formel (XXII)

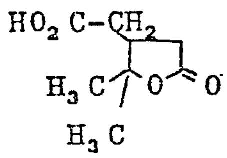

(XXII)

mit einem Chlorierungsmittel umgesetzt wird.

0017007

Europäisches Patentamt

# EUROPÄISCHER RECHERCHENBERICHT

Nummer der Anmeldung
EP 80102000.9

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int Cl ³) |
|---|---|---|---|
| | DE - A1 - 2 740 849 (BAYER) (22-03-1979)<br>+ Patentanspruch 1 +<br>-- | 5,8 | C 07 C 69/753<br>C 07 C 69/757<br>C 07 C 69/65<br>C 07 D 307/32<br>C 07 D 303/02<br>C 07 D 303/08<br>C 07 C 47/232<br>C 07 C 47/24<br>C 07 D 317/52<br>C 07 C 21/24<br>C 07 C 25/24<br>C 07 C 43/225 |
| D | DE - A1 - 2 738 150 (FMC CORP.)<br>+ Patentansprüche 1,13 +<br>-- | 1,14 | |
| | GB - A - 813 539 (BADISCHE ANILIN)<br>+ Patentanspruch 1 +<br>-- | 14 | |
| | AT - B - 283 320 (HOECHST)<br>+ Patentanspruch +<br>-- | 15 | RECHERCHIERTE SACHGEBIETE (Int. Cl. ³)<br>C 07 C 69/00<br>C 07 D 307/00<br>C 07 D 303/00<br>C 07 C 47/00<br>C 07 D 317/00<br>C 07 C 21/00<br>C 07 C 25/00<br>C 07 C 43/00 |
| P | DE - A1 - 2 827 101 (BAYER) (10-01-1980)<br>+ Patentanspruch +<br>& EP-A1-6 205 (09-01-1980)<br>-- | 1,14 | |
| | US - A - 4 157 447 (J.F. ENGEL)<br>+ Patentansprüche 1,2 +<br>-- | 1 | |
| | DE - A1 - 2 539 895 (SAGAMI)<br>+ Patentansprüche 1,2 +<br>---- | 1 | |

KATEGORIE DER GENANNTEN DOKUMENTE

- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| WIEN | 25-06-1980 | REIF |